# EUROPEAN PATENT APPLICATION

(11) **EP 4 492 038 A1**
(43) Date of publication of application: **15.01.2025**
(21) Application number: 24186810.8
(22) Date of filing: 05.07.2024
(51) Int. Cl.: G01N 15/14

(54) **METHOD FOR ANALYZING BLOOD CELLS AND REAGENT FOR BLOOD CELL ANALYSIS**

(30) Priority: 10.07.2023 JP 2023113305; 10.07.2023 JP 2023113309; 10.07.2023 JP 2023113311
(71) Applicant: SYSMEX CORPORATION, Kobe-shi Hyogo 651-0073 (JP)
(72) Inventor: MATSUBA, Hiroaki, Kobe-shi, Hyogo, 651-0073 (JP); TAKARA, Chihiro, Kobe-shi, Hyogo, 651-0073 (JP)
(74) Representative: Hoffmann Eitle

(57) **Abstract**

Disclosed is a method for analyzing blood cells, the method including:
acquiring first scattered light information, second scattered light information, and fluorescence information generated by irradiating a measurement sample that contains a particle stained with a fluorescent dye with a light of a first wavelength and a light of a second wavelength;
identifying blood cells other than white blood cell from the particles in the measurement sample, with reference to the first scattered light information and the second scattered light information, and/or, with reference to the first scattered light information and the fluorescence information; and
classifying the blood cells other than white blood cell into reticulocyte and platelet, with reference to the fluorescence information and the second scattered light information,
the first wavelength being 315 nm or longer and 600 nm or shorter, and the second wavelength being 610 nm or longer and 750 nm or shorter.

## Description

### FIELD OF THE INVENTION

The present invention relates to a method for analyzing blood cells. The present invention also relates to a reagent for blood cell analysis.

### BACKGROUND

Blood includes various blood cell components such as red blood cell, white blood cell, and platelet. Among them, the platelet is a non-nucleated cell having a diameter of 2 to 3 µm, whose count in normal blood is 150,000 to 350,000/µL. The platelet count has, however, been known to decrease in blood of patients with idiopathic thrombocytopenic purpura, acute leukemia and so forth. The platelet count in blood fallen below 10,000/µL is a widely-accepted criteria for need of blood transfusion. It is, therefore, important to exactly classify and count the platelet in clinical environment.

Blood also contains reticulocyte. The reticulocyte is a young red blood cell derived from erythroblastic cell in bone marrow, which is immediately after enucleated and released into peripheral blood. The reticulocyte usually becomes mature red blood cell in approximately one day. The reticulocyte contains a small amount of ribonucleic acid, and organelles such as ribosome and mitochondria, all of which are not found in mature red blood cell. Classification and counting of the reticulocyte are very important for determination of hematopoietic ability of bone marrow of subjects. For example, the reticulocyte count decreases in a depressed state of myelopoiesis, typically due to aplastic anemia, or chemotherapy for malignant tumor. Alternatively, the reticulocyte count increases in a hyperactive state of myelopoiesis, typically due to hemolytic anemia.

U.S. Patent Application Publication No. 2021/0041341 describes, in its EXAMPLE 8, measurement of reticulocyte and platelet, by preparing a sample with use of a dye for staining platelet and white blood cell, and a dye for staining reticulocyte, and by irradiating the sample with blue light (488 nm).

### SUMMARY OF THE INVENTION

White blood cell would affect the classification and counting of reticulocyte and platelet in some specimen. The prior method has, however, not paid a special attention on influence of white blood cell. It is therefore an object of the present invention to provide a means for classifying and counting reticulocyte and platelet, while suppressing the influence of white blood cell.

The present inventors have found that reticulocyte and platelet are detectable with use of fluorescence information, first scattered light information, and second scattered light information, which are obtainable by staining blood cells in a specimen with a fluorescent dye, and by irradiating the stained blood cells with light having a wavelength of 315 nm or longer and 600 nm or shorter, and light having a wavelength of 610 nm or longer and 750 nm or shorter. The inventors have thus completed the inventions according to Items [1] to [31] below.
[1] A method for analyzing blood cells, the method including:
   acquiring first scattered light information, second scattered light information, and fluorescence information generated by irradiating a measurement sample that contains a particle stained with a fluorescent dye with a light of a first wavelength and a light of a second wavelength;
   identifying blood cells other than white blood cell from the particles in the measurement sample, with reference to the first scattered light information and the second scattered light information, and/or, with reference to the first scattered light information and the fluorescence information; and
   classifying the blood cells other than white blood cell into reticulocyte and platelet, with reference to the fluorescence information and the second scattered light information,
   the first wavelength being 315 nm or longer and 600 nm or shorter, and the second wavelength being 610 nm or longer and 750 nm or shorter.
[2] The method according to [1], in which
   the first scattered light information is information related to side-scattered light generated from the particle upon irradiation of the measurement sample with the light of the first wavelength,
   the second scattered light information is information related to forward-scattered light generated from the particle upon irradiation of the measurement sample with the light having the second wavelength, and
   the fluorescence information is information related to fluorescence generated from the fluorescent dye on the particle upon irradiation of the measurement sample with the light of the first wavelength.
[3] The method according to [1] or [2], in which in the step of classifying, the blood cells other than white blood cell are classified into reticulocyte, platelet, and mature red blood cell.
[4] The method according to any one of [1] to [3], in which the first scattered light information is an intensity of first side-scattered light from the particle irradiated with the light of the first wavelength, the second scattered light information is an intensity of forward-scattered light from the particle irradiated with the light of the second wavelength, and the fluorescence information is an intensity of fluorescence from the fluorescent dye on the particle irradiated with the light of the first wavelength; and
   in the step of identifying, the blood cells other than white blood cell are identified, with reference to the first side-scattered light intensity, and the forward-scattered light intensity or the fluorescence intensity.
[5] The method according to [4], in which in the step of identifying, a scattergram is created, with reference to the first side-scattered light intensity, and the forward-scattered light intensity or the fluorescence intensity, and the blood cells other than white blood cell are identified on the scattergram.
[6] The method according to [4] or [5], in which in the step of identifying:
   the particles in the measurement sample are classified into a first particle population and a second particle population, with reference to the first side-scattered light intensity and the forward-scattered light intensity; and,
   the second particle population is a population with higher first side-scattered light intensity and/or the forward-scattered light intensity than the first particle population,
   the first particle population is identified as the blood cells other than white blood cell.
[7] The method according to [4] or [5], in which in the step of identifying:
   the particles in the measurement sample are classified into a first particle population and a second particle population, with reference to the first side-scattered light intensity and the fluorescence intensity; and,
   the second particle population is a population with higher first side-scattered light intensity and/or the fluorescence intensity than the first particle population,
   the first particle population is identified as the blood cells other than white blood cell.
[8] The method according to any one of [1] to [7], in which the second scattered light information is an intensity of the forward-scattered light from the particle irradiated with the light of the second wavelength, the fluorescence information is an intensity of the fluorescence from the particle irradiated with the light of the first wavelength, and
   in the step of classifying, the blood cells other than white blood cell are classified into reticulocyte and platelet, with reference to the fluorescence intensity and the forward-scattered light intensity.
[9] The method according to [8], in which in the step of classifying, a scattergram is created with reference to the fluorescence intensity and the forward-scattered light intensity, and the blood cells other than white blood cell are classified into reticulocyte and platelet, on the scattergram.
[10] The method according to [8] or [9], in which in the step of classifying:
   the blood cells other than white blood cell are classified into a third particle population, a fourth particle population, and a fifth particle population, with reference to the fluorescence intensity and the forward-scattered light intensity; and,
   the third particle population is a population with higher fluorescence intensity than the fifth particle population, and
   the fourth particle population is a population with lower forward-scattered light intensity than the third particle population,
   the third particle population is identified as a reticulocyte population, and the fourth particle population as a platelet population.
[11] The method according to [10], in which the fifth particle population is identified as a mature red blood cell population.
[12] The method according to any one of [1] to [11], in which in the step of identifying, white blood cell is further identified, and the white blood cell is classified into subpopulations.
[13] The method according to [12], in which the white blood cell is identified, with reference to the first scattered light information and the second scattered light information, and/or, with reference to the first scattered light information and the fluorescence information, and the white blood cell is classified into subpopulations.
[14] The method according to [13], in which the first scattered light information is an intensity of the first side-scattered light from the particle irradiated with the light of the first wavelength, the second scattered light information is an intensity of the forward-scattered light from the particle irradiated with the light of the second wavelength, and the fluorescence information is an intensity of fluorescence from the fluorescent dye on the particle irradiated with the light of the first wavelength; and
   the white blood cell is identified with reference to the first side-scattered light intensity, and the forward-scattered light intensity or the fluorescence intensity, and is classified into subpopulations.
[15] The method according to [14], in which a scattergram with reference to the first side-scattered light intensity and the forward-scattered light intensity, and/or, a scattergram with reference to the first side-scattered light intensity and the fluorescence intensity is prepared and the white blood cell is identified and classified into on the subpopulations on the scattergram.
[16] The method according to [12], in which in the step of acquiring, a third scattered light information is further acquired; and
   the third scattered light information is information related to a side-scattered light generated from the particle upon irradiation of the measurement sample with the light of the second wavelength,
   the white blood cell is identified with reference to the first scattered light information and the second scattered light information, and/or, with reference to the first scattered light information and the fluorescence information; and
   the white blood cell is classified into subpopulations with reference to the second scattered light information and the third scattered light information, and/or, with reference to the fluorescence information and the third scattered light information.
[17] The method according to [16], in which the second scattered light information is an intensity of the forward-scattered light from the particle irradiated with the light of the second wavelength, the third scattered light information is an intensity of the second side-scattered light from the particle irradiated with the light of the second wavelength, and the fluorescence information is an intensity of fluorescence from the fluorescent dye on the particle irradiated with the light of the first wavelength; and
   the white blood cell is classified into subpopulations, with reference to the second side-scattered light intensity, and the forward-scattered light intensity or the fluorescence intensity.
[18] The method according to [17], in which a scattergram is created with reference to the second side-scattered light intensity and the forward-scattered light intensity, and/or, a scattergram is created with reference to the second side-scattered light intensity and the fluorescence intensity, and the white blood cell is classified into subpopulations on the scattergram.
[19] The method according to any one of [12] to [18], in which the subpopulations includes at least two populations selected from lymphocyte population, monocyte population, and granulocyte population.
[20] The method according to any one of [1] to [19], in which the identifying further includes acquiring information that indicates presence or absence of platelet aggregation, with reference to the first scattered light information and the second scattered light information.
[21] The method according to [20], in which the first scattered light information is the intensity of the side-scattered light from the particle irradiated with the light of the first wavelength, the second scattered light information is the intensity of the forward-scattered light from the particle irradiated with the light of the second wavelength; and
   in the step of identifying, the blood cells other than white blood cell are identified, with reference to the forward-scattered light intensity and the side-scattered light intensity,
   the blood cells other than white blood cell are classified into a red blood cell-containing population and a platelet-containing population, and,
   information that indicates presence of the platelet aggregation is acquired, if the platelet-containing population contains a particle that exhibits a forward-scattered light intensity equal to or above a threshold value.
[22] The method according to [21], in which in the step of identifying, a scattergram is created with reference to the forward-scattered light intensity and the side-scattered light intensity; the particles in the measurement sample are identified as a red blood cell-containing population, a platelet-containing population, and white blood cell on the scattergram; and
   information that indicates presence of the platelet aggregation is acquired, if the platelet-containing population contains a particle that exhibits a forward-scattered light intensity equal to or above a threshold value.
[23] The method according to any one of [1] to [22], in which in the step of classifying, the platelet is further classified into mature platelet and immature platelet.
[24] The method according to any one of [1] to [23], in which in the step of classifying, a large platelet is identified from the platelet.
[25] The method according to any one of [1] to [24], further including estimating a platelet distribution width and/or a mean platelet volume.
[26] The method according to any one of [1] to [25], in which the fluorescent dye is selected from the group consisting of acridine-based compound, cyanine-based compound, and styryl-based compound.
[27] The method according to [26], in which the acridine-based compound is represented by formula (I) below: 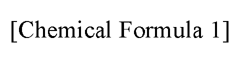 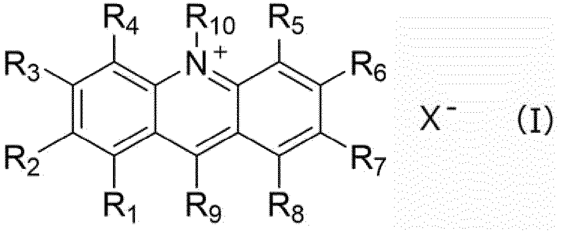 (where, each of R₁ to R₁₀ independently is a hydrogen atom, -NH₂, -(CH₂)ₙ-NR₁₁R₁₂, - NH-R₁₃-NR₁₁R₁₂, -O-R₁₁, -COOH, a halogen, a phenyl group optionally having a substituent, an alkyl group having 1 to 18 carbon atoms, or an acylamino group, provided that at least one of R₃, R₆, and R₉ independently is -NH₂, -(CH₂)ₙ-NR₁₁R₁₂, or phenyl group substituted with -NH₂;
   each of R₁₁ and R₁₂, identically or differently, is a hydrogen atom or alkyl group having 1 to 18 carbon atoms;
   R₁₃ is an alkyl group having 1 to 6 carbon atoms;
   n is an integer of 0 to 6; and
   X⁻ is a counter ion).
[28] The method according to [26] or [27], in which the cyanine-based compound is represented by formula (II) below: 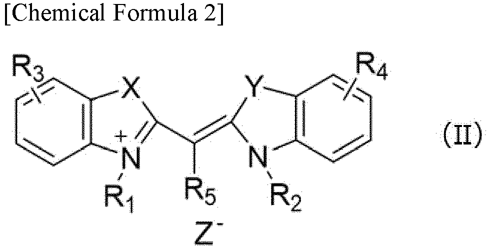 (where, each of R₁ and R₂, identically differently, is an alkyl group having 1 to 18 carbon atoms, an alkenyl group having 3 to 20 carbon atoms, an alkyl group having 1 to 18 carbon atoms and a hydroxy group, an alkyl group having 1 to 18 carbon atoms and a carboxy group, an alkyl group having 1 to 18 carbon atoms and a sulfo group, halogen, -(CH₂)ₘ-NR₆R₇, -(CH₂)ₙ-O-R₈, or a benzyl group optionally having a substituent;
   each of R₃ and R₄, identically differently, is a hydrogen atom, a halogen, an alkyl group having 1 to 18 carbon atoms, a phenyl group optionally having a substituent, an alkoxy group having 1 to 6 carbon atoms, or -(CH₂)ₙ-O-R₈;
   R₅ is a hydrogen atom, a halogen, an alkyl group having 1 to 6 carbon atoms, an alkyl group having 1 to 6 carbon atoms and a hydroxy group, an alkyl group having 1 to 6 carbon atoms and a carboxy group, an alkyl group having 1 to 6 carbon atoms and a sulfo group, a phenyl group optionally having a substituent, a benzyl group optionally having a substituent, or -(CH₂)ₙ-O-R₆;
   each of R₆ and R₇, identically or differently, is a hydrogen atom, or an alkyl group having 1 to 18 carbon atoms;
   R₈ is a hydrogen atom, an alkyl group having 1 to 18 carbon atoms, or a phenyl group optionally having a substituent;
   m is an integer of 1 to 18, and n is an integer of 1 to 6;
   each of X and Y, identically or differently, is a sulfur atom, an oxygen atom, a selenium atom, or CR₉R₁₀;
   each of R₉ and R₁₀, identically or differently, is an alkyl group having 1 to 3 carbon atoms; and
   Z⁻ is a counter ion).
[29] The method according to any one of [26] to [28], in which the styryl-based compound is represented by formula (III) below: 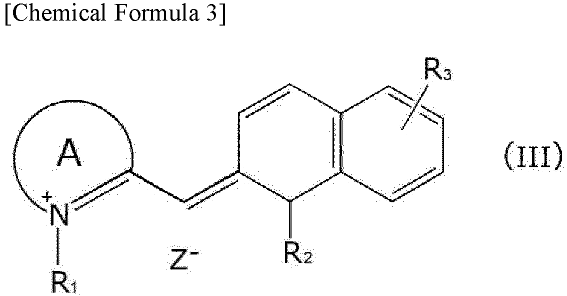 (where, R₁ is an alkyl group having 1 to 18 carbon atoms, an alkenyl group having 3 to 20 carbon atoms, an alkyl group having 1 to 18 carbon atoms and a hydroxy group, an alkyl group having 1 to 18 carbon atoms and a carboxy group, an alkyl group having 1 to 18 carbon atoms and a sulfo group, a halogen, -(CH₂)ₚ-NR₅R₆, -(CH₂)_{q}-O-R₇, or a benzyl group optionally having a substituent;
   each of R₂ and R₃, identically or differently, is a hydrogen atom, a halogen, an alkyl group having 1 to 6 carbon atoms, an alkyl group having 1 to 18 carbon atoms, a phenyl group optionally having a substituent, or an alkoxy group having 1 to 6 carbon atoms; 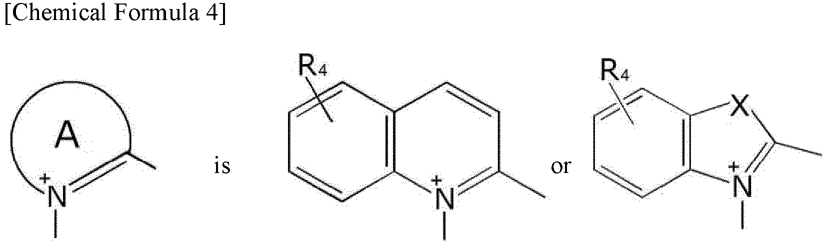
   R₄ is a hydrogen atom, a halogen, an alkyl group having 1 to 18 carbon atoms, a phenyl group optionally having a substituent, an alkoxy group having 1 to 6 carbon atoms, or -(CH₂)ₘ-O-R₇;
   each of R₅ and R₆, identically or differently, is a hydrogen atom, or an alkyl group having 1 to 18 carbon atoms;
   R₇ is a hydrogen atom, an alkyl group having 1 to 18 carbon atoms, or a phenyl group optionally having a substituent;
   X is a sulfur atom, an oxygen atom, a selenium atom, or CR₈R₉;
   each of R₈ and R₉, identically or differently, is an alkyl group having 1 to 3 carbon atoms;
   m is an integer of 1 to 18; and
   Z⁻ is a counter ion), or represented by formula (IV) below: 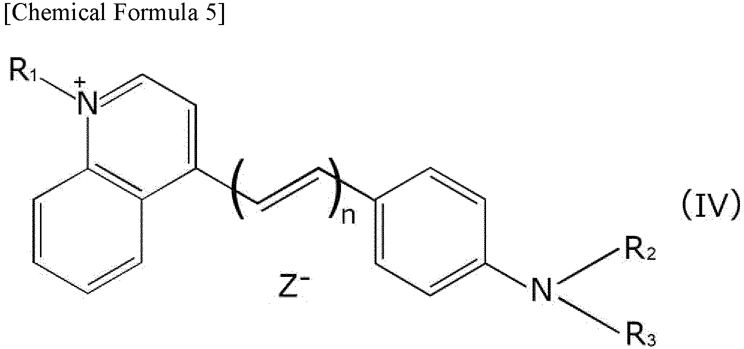 (where, R₁ and Z⁻ are same as those described above,
   each of R₂ and R₃, identically or differently, is a hydrogen atom, a halogen, an alkyl group having 1 to 18 carbon atoms, a phenyl group optionally having a substituent, or an alkoxy group having 1 to 6 carbon atoms, or, R₂ and/or R₃ forms an N-containing heterocycle together with a benzene ring to which the N is bound, and
   n is an integer of 0 to 2).
[30] The method according to any one of [1] to [29], in which the first wavelength is 315 nm or longer and 490 nm or shorter.
[31] A reagent for blood cell analysis, the reagent including a fluorescent dye selected from the group consisting of acridine-based compound, cyanine-based compound, and styryl-based compound, and used for the method according to any one of [1] to [30].

The present invention can provide a method for analyzing blood cells that enables classification and counting of reticulocyte and platelet, while suppressing influence of white blood cell, and a reagent for blood cell analysis for use in this method.

### BRIEF DESCRIPTION OF THE DRAWINGS

Fig. 1 is a perspective view illustrating an analysis system suitable for the method for analyzing blood cells of this embodiment.
Fig. 2 is a drawing illustrating a fluid circuit in a measurement unit, which involves a specimen suction unit, a sample preparation unit, and a flow cytometer (FCM) detector.
Fig. 3 is a block diagram illustrating a structure of the measurement unit.
Fig. 4 is a schematic drawing illustrating an exemplary structure of an optical system of the FCM detector.
Fig. 5 is a schematic drawing illustrating various kinds of light emitted from a particle that passes through a flow cell under irradiation with light, received by the optical system of the FCM detector.
Fig. 6 is a block diagram illustrating a structure of an analysis unit.
Fig. 7 is a flowchart illustrating a flow of operations of the analysis system.
Fig. 8 is a flowchart illustrating procedures of a measurement process.
Fig. 9 is a drawing illustrating an exemplary reagent for blood cell analysis of this embodiment.
Fig. 10A is a flowchart illustrating procedures of an analysis process in a first embodiment.
Fig. 10B is a flowchart illustrating procedures of the analysis process in the first embodiment.
Fig. 10C is a flowchart illustrating procedures of the analysis process in the first embodiment.
Fig. 10D is a flowchart illustrating procedures of the analysis process in the first embodiment.
Fig. 11 is a flowchart illustrating a modified example of a classification process of white blood cell.
Fig. 12 is a schematic drawing of a first scattergram (a), with a first side-scattered light (SSC-1) intensity plotted on the abscissa, and with a second forward-scattered light (FSC-2) intensity plotted on the ordinate, exemplifying positions where a first particle population and a second particle population appear.
Fig. 13A is a schematic drawing of the first scattergram (a), exemplifying positions where an RBC-containing population, a PLT-containing population, and the second particle population appear.
Fig. 13B is a schematic drawing of the first scattergram (a), exemplifying a position where platelet aggregation appears.

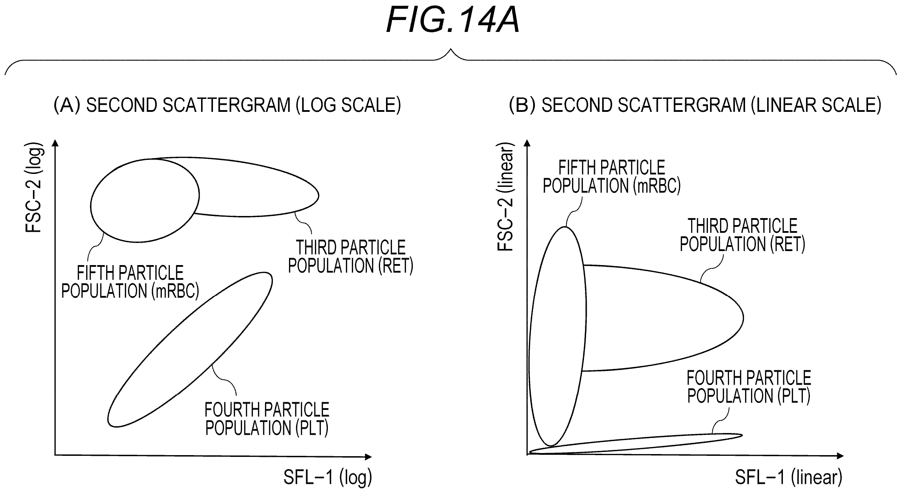

Fig. 14A contains schematic drawings, where (A) is a second scattergram (in logarithmic scale), with a logarithm of the first side-fluorescence (SFL-1) intensity (SFL-1(log)) plotted on the abscissa, and with a logarithm of the second forward-scattered light intensity (FSC-2(log)) plotted on the ordinate, exemplifying positions where a third particle population, a fourth particle population, and a fifth particle population appear; and (B) is a second scattergram (in linear scale), with the SFL-1 intensity plotted on the abscissa, and with the FSC-2 intensity plotted on the ordinate, exemplifying positions where the third particle population, the fourth particle population, and the fifth particle population appear.
Fig. 14B contains schematic drawings, where (A) is the second scattergram (in logarithmic scale), exemplifying areas where mRBC and RET individually appear; and (B) is the second scattergram (in linear scale), exemplifying areas where mRBC and RET individually appear.
Fig. 15A contains schematic drawings, where (A) is the second scattergram (in logarithmic scale), exemplifying an area where immature platelet appears; (B) is the second scattergram (in logarithmic scale), exemplifying an area where large platelet appears; (C) is the second scattergram (in logarithmic scale), exemplifying an area where large platelet appears; and (D) is an exemplary histogram created with reference to FSC-2 intensity and count of sorted platelet. In the drawings, "MPV" stands for mean platelet volume, and "PDW" stands for platelet distribution width.
Fig. 15B is a schematic drawing of the second scattergram (in linear scale), exemplifying an area where mRBC appears, a low fluorescence rate (LFR) area, a middle fluorescence rate (MFR) area, and a high fluorescence rate (HFR) area.
Fig. 16 is a schematic drawing of the first scattergram (a), exemplifying areas where the individual subpopulations of white blood cell appear.
Fig. 17 contains schematic drawings, where (A) is a third scattergram (a), with the second side-scattered light (SSC-2) intensity plotted on the abscissa, and with the FSC-2 intensity plotted on the ordinate, exemplifying positions where the individual subpopulations of white blood cell appear; and (B) is a third scattergram (b), with the SSC-2 intensity plotted on the abscissa, and with the SFL-1 intensity plotted on the ordinate, exemplifying positions where the individual subpopulations of white blood cell appear.
Fig. 18 is a flowchart illustrating procedures of analysis in a second embodiment.
Fig. 19 is a schematic drawing of the first scattergram (b), with the SSC-1 intensity plotted on the abscissa, and with the SFL-1 intensity plotted on the ordinate, exemplifying positions where the first particle population and the second particle population appear.
Fig. 20 is a schematic drawing of the first scattergram (b), exemplifying positions where the individual subpopulations of white blood cell appear.
Fig. 21A contains the first scattergram (a) and the second scattergram of high-RET% specimen 1 of Example 1.
Fig. 21B contains the first scattergram (b) and the second scattergram of high-RET% specimen 1 of Example 1.
Fig. 22A contains the first scattergram (a) and the second scattergram of low-RET% specimen 1 of Example 1.
Fig. 22B contains the first scattergram (b) and the second scattergram of low-RET% specimen 1 of Example 1.
Fig. 23A contains the first scattergram (a) and the second scattergram of high-RET% specimen 2 of Example 2.
Fig. 23B contains the first scattergram (b) and the second scattergram of high-RET% specimen 2 of Example 2.
Fig. 24A contains the first scattergram (a) and the second scattergram of low-RET% specimen 2 of Example 2.
Fig. 24B contains the first scattergram (b) and the second scattergram of low-RET% specimen 2 of Example 2.
Fig. 25A contains the first scattergram (a) and the second scattergrams of low-PLT specimen 1 stained with 3-ethyl-2-[(3-ethyl-2-benzothiazolinylidene)methyl]benzothiazolium in Example 3.
Fig. 25B contains the first scattergram (b) and the second scattergrams of low-PLT specimen 1 stained with 2,7,9-trimethylacridine-3,6-diamine in Example 3.
Fig. 26A contains the first scattergrams (a) and the second scattergrams of low-PLT specimens 1 stained with various fluorescent dyes in Example 3.
Fig. 26B contains the first scattergrams (b) and the second scattergrams of low-PLT specimens 1 stained with various fluorescent dyes in Example 3.
Fig. 27A contains the first scattergrams (a) and the second scattergrams of low-PLT specimens 2 stained with various fluorescent dyes in Example 3.
Fig. 27B contains the first scattergrams (b) and the second scattergrams of low-PLT specimens 2 stained with various fluorescent dyes in Example 3.
Fig. 28 contains the first scattergrams (a) and comparative scattergrams of a PLTc-negative specimen and a PLTc-positive specimen in Example 4.
Fig. 29 contains the first scattergrams (a) and the third scattergram (a) of blood samples from healthy persons in Example 5.
Fig. 30 contains the first scattergrams (b) and the third scattergram (b) of blood samples from healthy persons in Example 5.
Fig. 31 contains graphs illustrating white blood cell counts in normal specimens and abnormal specimens in Example 6, obtained from the first scattergrams (a) and (b), correlated with the counts obtained from prior methods (WDF channel and WNR channel).
Fig. 32 contains drawings, where (A) is the second scattergram (in logarithmic scale) of blood samples from healthy persons in Example 7; and (B) is a histogram for the sorted platelet.

### DETAILED DESCRIPTION OF THE PREFERRED EMBODIMENTS

### (Analysis System)

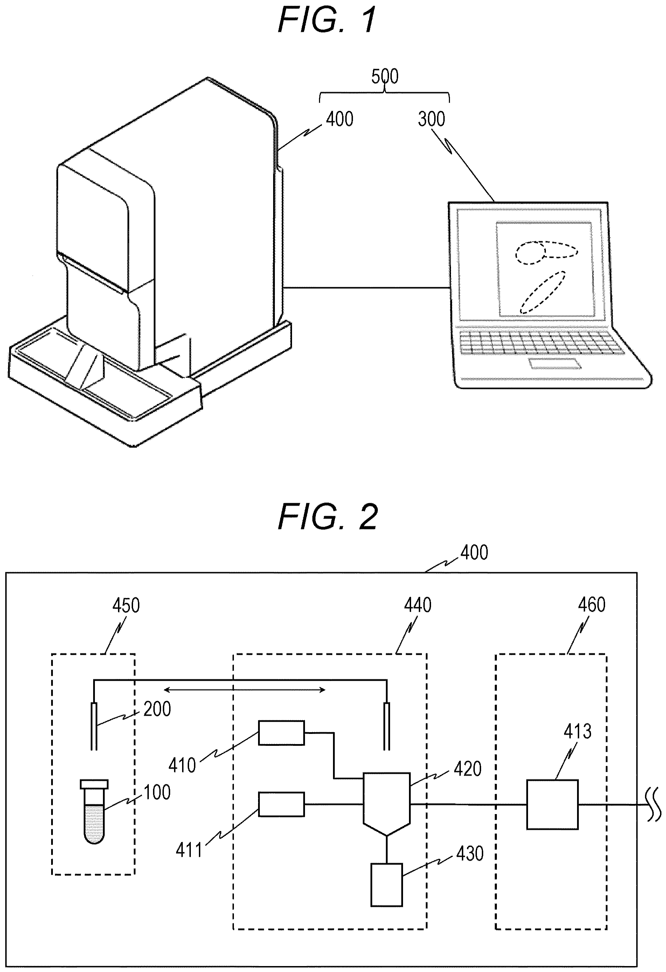

First, an analysis system suitable for a method for analyzing blood cells of this embodiment will be described, while referring to Fig. 1. An analysis system 500 has a measurement unit 400 as a measurement apparatus, and an analysis unit 300 as an analyzer. The analysis unit 300 is typically a personal computer having installed thereon software for analyzing specimen to be measured. The analysis unit 300 also takes part in operational control of the measurement unit 400. The analysis system 500 may alternatively be structured, with the analysis unit 300 incorporated in the measurement unit 400.

The measurement unit 400 is a unit for measuring the specimen, and contains a flow cytometer. In the measurement unit 400, the specimen and a reagent are mixed to prepare a measurement sample. The measurement sample is prepared with use of reagents, which are a staining reagent and a dilution reagent. The staining reagent contains a fluorescent dye for staining particle. The dilution reagent contains a buffering agent and a solvent. The specimen, the staining reagent, and the dilution reagent will be detailed later. In analysis of the particles in the specimen with this analysis system, particles stained with the fluorescent dye in the measurement sample are analyzed. The "particles in the measurement sample" refers to elements contained in the measurement sample, which can be individually measured by an FCM detector 460 described later. The particles in the measurement sample are exemplified by cell contained in the specimen, red blood cell ghosts, platelet aggregation, lipid particle, fungi, and bacteria. The term "cell" encompasses various types of blood cell and platelet. The red blood cell ghost refers to a debris of hemolyzed red blood cell. The red blood cell ghost may be produced also due to hemolysis caused by other than the hemolysis reagent, that is, caused by physical impact on blood collection tube; or low pH or low osmotic pressure of the measurement sample.

The measurement sample is measured with an FCM detector 460 (see Fig. 2), described later, in the measurement unit 400. From the measurement, acquired are an optical signal related to fluorescence emitted from the fluorescent dye on the stained particles in the measurement sample, and an optical signal related to scattered light emitted from the particle. The "fluorescent dye on the particle" refers to a fluorescent dye, which is bound to the particle by staining. Mode of binding between the particle and the fluorescent dye is not particularly limited, as long as the particle and the fluorescent dye are integrally measurable by the flow cytometer. The acquired optical signals are subjected to A/D conversion. Digital data is thus acquired. The analysis unit 300 analyzes the digital data obtained by the measurement unit 400, to identify and/or classify the particles in the measurement sample.

A structure of a fluid system in the measurement unit 400 will be described, while referring to Fig. 2. The measurement unit 400 contains a sample preparation unit 440, a specimen suction unit 450, and the FCM detector 460. The sample preparation unit 440 contains a reagent container 410, a reagent container 411, a reaction chamber 420, and a waste liquid chamber 430. For example, the reagent container 410 stores a dilution reagent, meanwhile the reagent container 411 stores a staining reagent. The reagent container 410 and the reagent container 411 are individually connected through a liquid feeding tube to the reaction chamber 420. In the sample preparation unit 440, the dilution reagent and the staining reagent are injected through the individual liquid feeding tubes into the reaction chamber 420. The specimen suction unit 450 contains a suction tube 200. The suction tube 200 sucks the specimen contained in the blood collection tube 100, and discharges the specimen into the reaction chamber 420. The reaction chamber 420 is connected through a liquid feeding tube, to a flow cell 413 of the FCM detector 460.

The reaction chamber 420 is a container for preparing a measurement sample. In the reaction chamber 420, mixed are the specimen, the staining reagent containing a fluorescent dye, and the dilution reagent, to prepare the measurement sample. The measurement sample in the reaction chamber 420 is fed through the liquid feeding tube to the flow cell 413 of the FCM detector 460, and measured. The FCM detector 460 acquires various optical signals emitted from each particle in the measurement sample. After completion of the measurement with the FCM detector 460, the measurement sample that remained in the reaction chamber 420 is discarded in the waste liquid chamber 430. The reaction chamber 420 is washed with an unillustrated washing mechanism, before preparation of the next measurement sample.

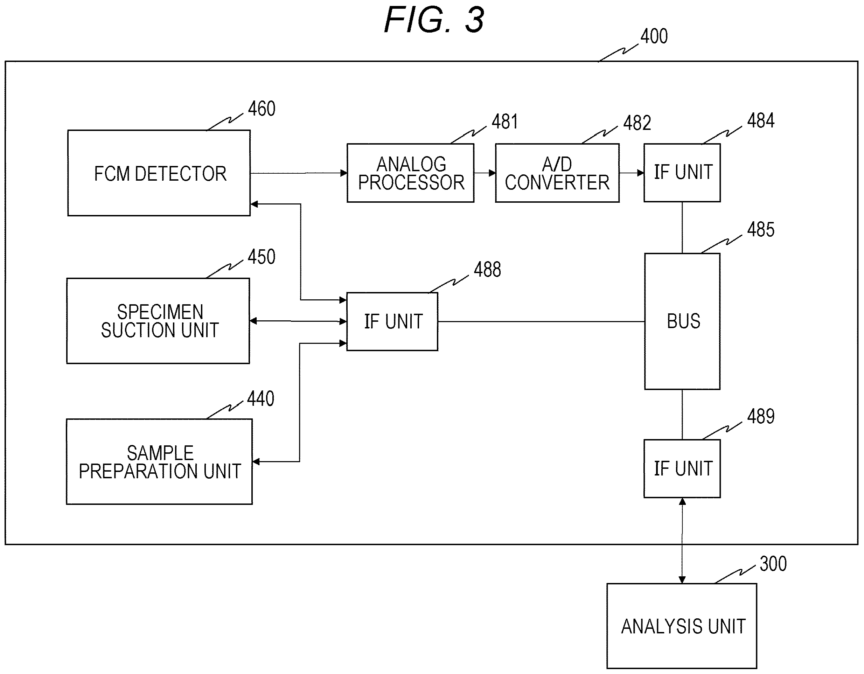

Electrical connection of the individual components in the measurement unit 400 will be described, while referring to Fig. 3. The sample preparation unit 440, the specimen suction unit 450, and the FCM detector 460 are connected to an interface (IF) unit 488. The FCM detector 460 is connected to an analog processor 481 and an A/D converter 482. Analog processor 481 processes an analog signal output from the FCM detector 460, and the A/D converter 482 converts the analog signal output from the analog processor 481 into a digital signal. The measurement unit 400 is connected through an IF unit 489 to the analysis unit 300. An IF unit 484, the IF unit 488, and the IF unit 489 are connected to a bus 485.

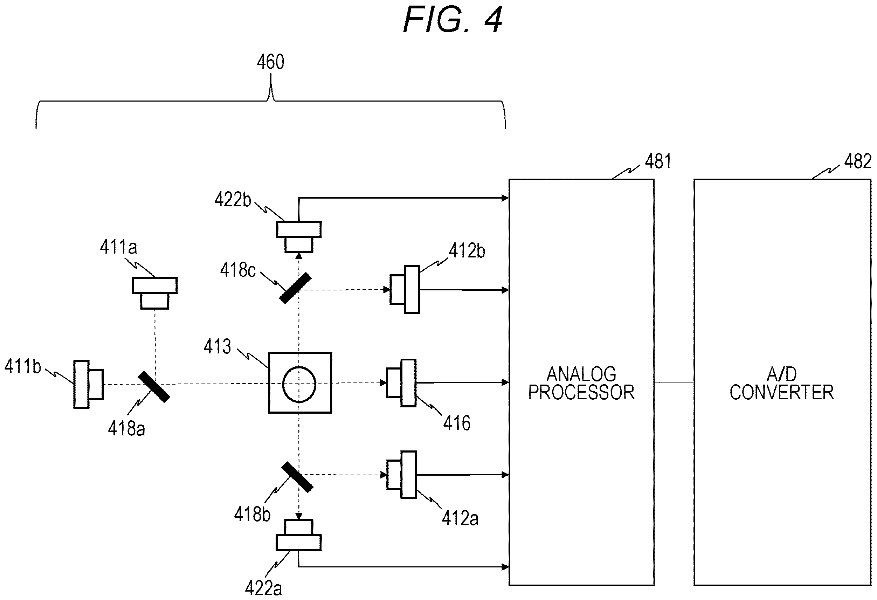

The FCM detector 460, the analog processor 481, and the A/D converter 482 will be described, while referring to Fig. 4. The FCM detector 460 has a first light source 411a, a second light source 411b, a flow cell 413, dichroic mirrors 418a, 418b, 418c, side-scattered light receivers 412a, 412b, a forward-scattered light receiver 416, and side-fluorescence receivers 422a, 422b. The first light source 411a and the second light source 411b emit light of different wavelengths. For example, the first light source 411a emits light of a first wavelength, and the second light source 411b emits light of a second wavelength. The first wavelength is preferably a wavelength that hemoglobin can absorb. Such wavelength typically falls in the range from 315 nm or longer to 600 nm or shorter, preferably 400 nm or longer and 490 nm or shorter, more preferably 400 nm or longer and 450 nm or shorter, and yet more preferably 400 nm or longer and 410 nm or shorter. The light in the aforementioned wavelength range, when irradiated on mature red blood cell or reticulocyte that contain hemoglobin, is absorbed by hemoglobin in these blood cells, so that parameters such as intensity (peak value), pulse width, and pulse area of scattered lights from the mature red blood cell or reticulocyte tend to be smaller than those from white blood cell that does not contain hemoglobin. The second wavelength is different from the first wavelength, whose wavelength is preferably not absorbable by hemoglobin. Such wavelength typically falls in the range from 610 nm or longer to 750 nm or shorter, preferably 620 nm or longer and 700 nm or shorter, and more preferably 633 nm or longer and 643 nm or shorter. Use of both lights, absorbable by hemoglobin and not absorbable by hemoglobin, is considered to clearly distinguish mature red blood cell or reticulocyte, from white blood cell. A semiconductor laser light source, for example, is applicable to the first and second light sources.

The measurement sample prepared in the reaction chamber 420 is fed to the flow cell 413 of the FCM detector 460. In the illustration of Fig. 4, the measurement sample flows in a direction perpendicular to this page. With the measurement sample kept flowed through the flow cell 413, light emitted from the first light source 411a is reflected on the dichroic mirror 418a, and then irradiated on each particle in the measurement sample that flows through the flow cell 413. Light emitted from the second light source 411b transmits through the dichroic mirror 418a, and then irradiated on each particle in the measurement sample that flows through the flow cell 413.

In the example of Fig. 4, the forward-scattered light receiver 416 is arranged so as to receive forward-scattered light emitted from the particle upon being irradiated with the light from the second light source 411b. The light receiver 416 may alternatively be arranged so as to receive the forward-scattered light emitted from the particle upon being irradiated with the light from the first light source 411a. Still alternatively, an additional light receiver may be arranged so as to receive the forward-scattered light emitted from the particle upon being irradiated with the light from the first light source 411a. The forward-scattered light is typically a scattered light which may be received at a receiving angle of 0 to approximately 20 degrees. The forward-scattered light receiver 416 is typically a photodiode. The side-scattered light (first side-scattered light) that corresponds to the light irradiated by the first light source 411a is reflected on the dichroic mirror 418b, and then received by the side-scattered light receiver 412a. The side-scattered light (second side-scattered light) corresponded to the light irradiated by the second light source 411b is reflected on the dichroic mirror 418c, and then received by the side-scattered light receiver 412b. The side-scattered light is typically a scattered light which may be received at a receiving angle of approximately 20 degrees to approximately 90 degrees. The side-scattered light receivers 412a and 412b each are typically a photodiode.

The method for analyzing blood cells of this embodiment uses a fluorescent dye that can be excited at the first wavelength. The side-fluorescence (first side-fluorescence) that corresponds to the light emitted upon excitation of the fluorescent dye transmits through the dichroic mirror 418b, and then received by the side-fluorescence receiver 422a. Although the method for analyzing blood cells of this embodiment does not use any fluorescent dye that can be excited at the second wavelength, the example of Fig. 4 illustrates a structure capable of receiving the side-fluorescence (second side-fluorescence) that corresponds to the light emitted upon excitation of the fluorescent dye, for the convenience of explaining the optical system. That is, the second side-fluorescence transmits through dichroic mirror 418c, and then received by the side-fluorescence receiver 422b. The side-fluorescence receivers 422a and 422b each is typically an avalanche photodiode.

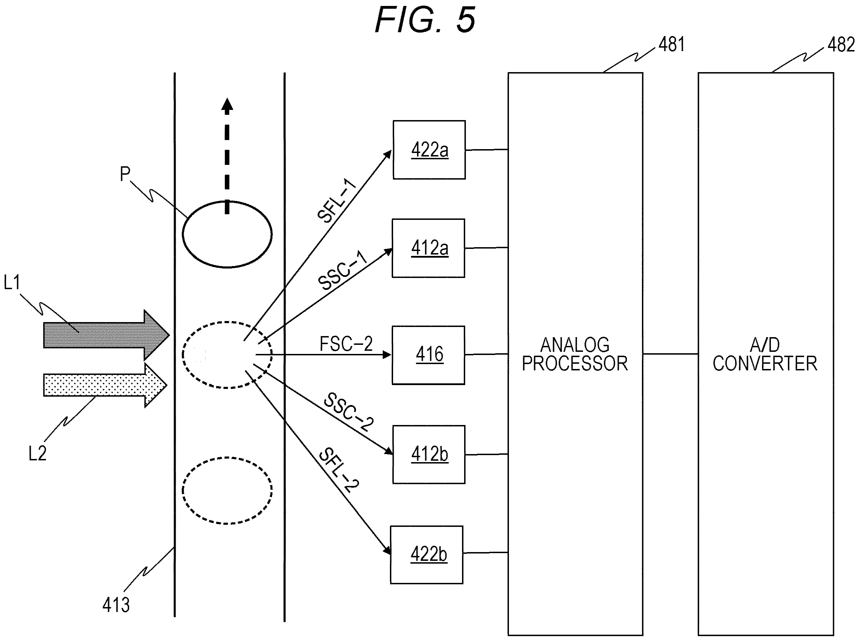

Relations of the various types of light emitted from a particle P that passes through the flow cell 413, with the optical system of the FCM detector 460 will be explained, while referring to Fig. 5. In Fig. 5, the light emitted from the first light source 411a is light L1 of the first wavelength, and the light emitted from the second light source 411b is light L2 of the second wavelength. Upon being irradiated with lights L1 and L2, the particle P that passes through the flow cell 413 emits forward-scattered lights that individually correspond to the light of the first wavelength and the light of the second wavelength, ahead of the direction of travel of light. Since the light receiver 416 herein receives the forward-scattered light that corresponds to the light of the second wavelength, Fig. 5 illustrates only the second forward-scattered light (FSC-2) that corresponds to the light of the second wavelength, while omitting the first forward-scattered light (FSC-1) that corresponds to the light of the first wavelength. Referring now to Fig. 5, there are generated the first side-scattered light (SSC-1) that corresponds to light of the first wavelength, and the second side-scattered light (SSC-2) that corresponds to the light of the second wavelength, emitted laterally with respect to the direction of travel of light. With the particle P stained with the fluorescent dye that can be excited with the light of the first wavelength, obtainable is the first side-fluorescence (SFL-1) excited with the light of the first wavelength, emitted laterally with respect to the direction of travel of light. Although the method for analyzing blood cells of this embodiment does not use any fluorescent dye that can be excited at the second wavelength, the example of Fig. 5 illustrates a state in which both of SFL-1, and the second side-fluorescence (SFL-2) excited by the light of the second wavelength are generated, laterally to the direction of travel of light, for the convenience of explaining the optical system.

As has been described, FSC-2, SSC-1, SFL-1, SSC-2, and SFL-2 are received by the light receivers 416, 412a, 422a, 412b, and 422b, respectively. Each light receiving element outputs a wavy electric signal (also referred to as an analog signal) that contains a pulse corresponded to the received light intensity. The analog signal corresponded to FSC-2 is also referred to as the "second forward-scattered light signal", the analog signal corresponded to the SSC-1 also as the "first side-scattered light signal", the analog signal corresponded to SFL-1 also as the "first fluorescence signal", the analog signal corresponded to SSC-2 also as the "second side-scattered light signal", and the analog signal corresponded to SFL-2 also as the "second fluorescence signal". In a case where FSC-1 is received, the analog signal corresponded to FSC-1 is also referred to as the "first forward-scattered light signal". One pulse of each analog signal corresponds to one particle (one cell, for example).

The analog signals corresponded to the various types of light are individually input to the analog processor 481, and subjected to processes such as noise removal and smoothing. The A/D converter 482 samples analog signals output from the analog processor 481, at a predetermined sampling rate (for example, 1024 points sampled at 10 nanosecond intervals, or 128 points sampled at 80 nanosecond intervals, or 64 points sampled at 160 nanosecond intervals). The A/D converter 482 digitizes the sampled analog signal, to produce waveform data. The A/D converter 482 samples and digitizes five types of analog signals that correspond to the individual cells that flow through the flow cell 413, to create waveform data including the second forward-scattered light signal, the first side-scattered light signal, the first fluorescence signal, the second side-scattered light signal, and the second fluorescence signal. In a case where FSC-1 is received, also waveform data of the first forward-scattered light signal is created. The A/D converter 482 also calculates a feature parameter that represents a morphological feature of each cell, from the waveform data of each signal. Such feature parameter is exemplified by peak value (height of pulse peak), pulse width, pulse area, transmittance, Stokes shift, ratio, temporal change, and values correlated thereto.

Optical information can be the aforementioned feature parameter. The optical information includes fluorescence information, first scattered light information, second scattered light information, and third scattered light information. The fluorescence information relates to the fluorescence generated from the fluorescent dye on the stained particle, upon irradiation of the measurement sample with light of the first wavelength. The fluorescence information is not particularly limited as long as it reflects the amount of the fluorescent dye that has stained the nucleic acid in nucleated cell. The fluorescence information is preferably peak value or pulse area of the first fluorescence signal, and is more preferably the peak value of the first fluorescence signal. The peak value of the first fluorescence signal herein is also referred to as "first fluorescence intensity". The first fluorescence intensity is understood to be intensity of fluorescence from the fluorescent dye on the particle irradiated with the light of the first wavelength.

The first scattered light information relates to the side-scattered light generated from the particle, upon irradiation of the measurement sample with the light of the first wavelength. The second scattered light information relates to the forward-scattered light generated from the particle, upon irradiation of the measurement sample with the light of the second wavelength. The third scattered light information relates to the side-scattered light generated from the particle, upon irradiation of the measurement sample with the light of the second wavelength. Information on the side-scattered light is not particularly limited as long as it reflects internal information such as complexity of cellular structure, granule characteristic, nuclear structure, and degree of segmentation. Information on the forward-scattered light is not particularly limited as long as it reflects size of the particle. The first scattered light information is preferably peak value or pulse area of the first side-scattered light signal, and is more preferably the peak value of the first side-scattered light signal. The second scattered light information is preferably peak value or pulse area of the second forward-scattered light signal, and is more preferably the peak value of the second forward-scattered light signal. The third scattered light information is preferably peak value or pulse area of the second side-scattered light signal, and is more preferably the peak value of the second side-scattered light signal. The peak value of the first side-scattered light signal is also referred to as "first side-scattered light intensity", the peak value of the second forward-scattered light signal is also referred to as "second forward-scattered light intensity", and the peak value of the second side-scattered light signal is also referred to as "second side-scattered light intensity". The first side-scattered light intensity is understood to be intensity of the first side-scattered light emitted from the particle irradiated with the light of the first wavelength. The second forward-scattered light intensity is understood to be intensity of the forward-scattered light emitted from the particle irradiated with the light of the second wavelength. The second side-scattered light intensity is understood to be intensity of the second side-scattered light emitted from the particle irradiated with the light of the second wavelength.

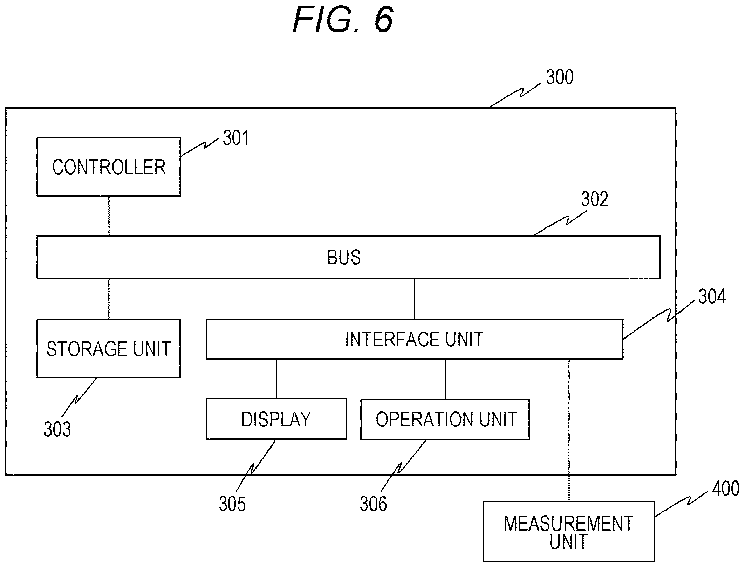

Referring now to Fig. 6, the analysis unit 300 is electrically connected through the interface unit 304 to the measurement unit 400. The interface unit 304 is typically an USB interface. The analysis unit 300 contains a controller 301, a bus 302, a storage unit 303, an interface unit 304, a display 305, and an operation unit 306. The analysis unit 300 is typically constituted by a personal computer (see the analysis unit 300 in Fig. 1), and executes a program product stored in the storage unit 303, to control the measurement unit 400 of the analysis system 500. The analysis unit 300 analyzes data that includes the optical information acquired by the measurement unit 400, and displays an analysis result on the display 305. The analysis unit 300 may also classify the cells with reference to the optical information.

The storage unit 303 typically stores a program product for controlling the measurement unit 400, and a program product for analyzing data acquired by the measurement unit 400. The display 305 typically displays result of analysis of data acquired by the measurement unit 400. The operation unit 306 has a keyboard, and a pointing device that contains a mouse or a touch panel.

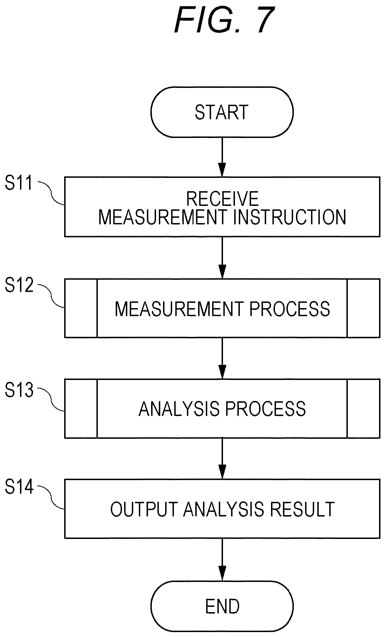

Exemplary operations of the analysis system 500 will now be described while referring to Fig. 7, without limiting the invention. In step S 11, the controller 301 of the analysis unit 300 receives a measurement start instruction from the user, through the operation unit 306. Upon receiving the measurement start instruction, the controller 301 transmits instruction data for instructing the measurement start to the measurement unit 400. The measurement unit 400 receives the instruction data, and executes a measurement process in step S12.

The method for analyzing blood cells of this embodiment typically encompasses a first embodiment and a second embodiment below. In the "first embodiment", a measurement sample prepared from a specimen is measured, to acquire the first scattered light information and the second scattered light information of the particles in the measurement sample. From among the particles, blood cells other than white blood cell, and white blood cell are then identified, with reference to the first scattered light information and the second scattered light information. From among the blood cells other than white blood cell, then classified are reticulocyte and platelet, with reference to the fluorescence information and the second scattered light information. Meanwhile in the "second embodiment", a measurement sample prepared from a specimen is measured, to acquire the first scattered light information and the fluorescence information of the particles in the measurement sample. From among the particles, blood cells other than white blood cell, and white blood cell are then identified, with reference to the first scattered light information and the fluorescence information. From among the blood cells other than white blood cell, then classified are reticulocyte and platelet, in the same manner as in the first embodiment.

### (Measurement Process)

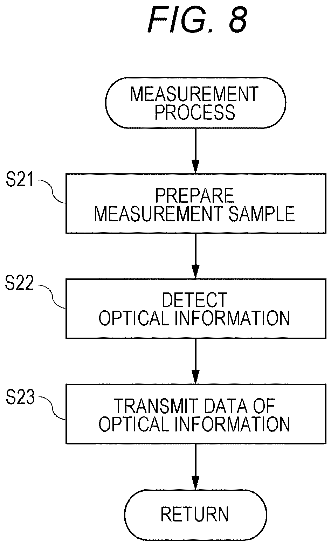

Exemplary measurement process in step S12 in Fig. 7 according to the first and second embodiments will be explained while referring to Fig. 8, without limiting the invention. In step S21, the controller 301 of the analysis unit 300 instructs the measurement unit 400 to prepare the measurement sample from the specimen. More specifically, the measurement unit 400 mixes the specimen, the staining reagent, and the dilution reagent in the reaction chamber 420, to prepare the measurement sample. In step S22, the controller 301 instructs the measurement unit 400 to feed the measurement sample to the FCM detector 460, and to implement optical measurement by flow cytometry. The measurement unit 400 thus detects optical information of each particle in the measurement sample, typically including waveform data of SSC-1, SSC-2, FSC-2, and SFL-1. In step S23, the controller 301 instructs the measurement unit 400 to transmit the optical information to the analysis unit 300. The measurement process thus finishes. The analysis unit 300 then implements the analysis process in step S13 in Fig. 7. Exemplary analysis processes of the individual embodiments will be described later.

### (Specimen)

The specimen is blood specimen. The blood specimen is exemplified by whole blood, and dilution of the whole blood. The whole blood is typically peripheral blood collected from the subject. The blood specimen may contain an anticoagulant. The anticoagulants is exemplified by ethylenediaminetetraacetate (EDTA), EDTA salts (EDTA 2K, EDTA 2Na, for example), sodium citrate, heparin, and warfarin. The dilution of whole blood is obtainable typically by diluting the whole blood with a suitable aqueous solvent, preferably with a dilution reagent described later. The aqueous solvent is exemplified by water, physiological saline, and aqueous solution of buffering agent.

The particles in the measurement sample are derived from blood components of the specimen. As referred herein, the "blood components" encompass the elements having been known to reside in blood, and abnormal cell. The elements having been known to reside in blood are typically blood cells usually contained in peripheral blood of healthy person. Such blood cells are exemplified by white blood cell, mature red blood cell, reticulocyte, and platelet. The "mature red blood cell" is a terminally differentiated red blood cell having neither nucleus nor nucleic acid. The term "mature red blood cell" herein is used for distinguishing this cell from reticulocyte. The mature red blood cell and reticulocyte, if not needed to be distinguished herein, may be collectively referred to as "red blood cell" or "RBC". The term "platelet" herein encompasses immature platelet and large platelet. "Immature platelet", also referred to as reticulated platelet, is a young platelet that has just been released into the peripheral blood. The mature platelet does not contain nucleic acid, whereas the immature platelet contains a small amount of ribonucleic acid. The "large platelet" has a diameter of 4 µm or larger. The platelet is usually 2 to 3 µm in diameter. The "abnormal cell" refers to a cell that does not normally appear in blood or body fluid. The abnormal cell also encompasses non-cellular particle and microorganism. The non-cellular particle is exemplified by platelet aggregation, red blood cell ghost, and lipid particle. The microorganism is exemplified by bacteria and fungi. The platelet aggregation occurs in a blood collection tube, due to contamination with tissue fluid during blood collection, insufficient mixing, or action of EDTA.

### (Dilution Reagent)

The dilution reagent used for preparing the measurement sample will be described. The dilution reagent contains a buffering agent and a solvent. The dilution reagent preferably contains a solution of the buffering agent. Preferred example of the solvent includes water and physiological saline. Water is particularly preferred. The buffering agent preferably demonstrates a buffering function within a pH range from 6 or higher and 11 or lower. Such buffers can be selected typically from, carboxylate, phosphate, Good's buffer, taurine, and triethanolamine. The dilution reagent typically has a pH of 6 or higher and 11 or lower, which is preferably 7 or higher and 10 or lower, and more preferably 8 or higher and 9.5 or lower. With the dilution reagent whose pH is 6 or above, red blood cell will be less likely to hemolyze in the measurement sample. This successfully suppresses red blood cell ghost from occurring. With the dilution reagent whose pH is 11 or below, red blood cells and red blood cell ghost may be prevented from being non-specifically stained with the fluorescent dye. A preferred dilution reagent is an aqueous solution of the buffering agent, having a pH of 6 or higher and 11 or lower.

The dilution reagent may further contain, besides the buffering agent, components such as osmotic pressure compensator, staining accelerator, highly-charged anion, and preservative. The osmotic pressure compensator is a substance capable of maintaining osmotic pressure of the dilution reagent within an appropriate range. The osmotic pressure compensator is exemplified by alkali metal salt of organic acid such as propionic acid; saccharide such as glucose or mannose; alkali metal halide such as sodium chloride; and alkaline earth metal halide such as magnesium chloride. The osmotic pressure compensator may be used singly or in combination of two or more kinds thereof. The osmotic pressure compensator, when used, is added to the dilution reagent, so as to adjust the osmotic pressure of the dilution reagent to 150 mOsm/kg■H2O or higher and 600 mOsm/kg■H2O or lower, and more preferably to 200 mOsm/kg■H2O or more and mOsm/kg■H2O or lower.

The staining accelerator is a substance capable of promoting permeability of the fluorescent dye into the blood cells. The staining accelerator is exemplified by surfactant, which is preferably cationic surfactant. Quaternary ammonium salt type surfactant is particularly preferred as the cationic surfactant, which is exemplified by decyltrimethylammonium bromide (DTAB), lauryltrimethylammonium chloride (LTAC), octyltrimethylammonium bromide, cetyltrimethylammonium chloride, and myristyltrimethylammonium bromide. The cationic surfactant may be used singly or in combination of two or more kinds thereof.

The concentration of the cationic surfactant in the dilution reagent is not particularly limited as long as hemolysis of red blood cell may be suppressed. The concentration may be appropriately determined typically within the range from 50 ppm or higher and 20,000 ppm or lower. In a specific case of using DTAB, the concentration thereof in the dilution reagent is preferably 500 ppm or higher and 3000 ppm or lower. In a case of using LTAC, the concentration thereof in the dilution reagent is preferably 100 ppm or higher and 500 ppm or lower. With such concentration of the cationic surfactant contained therein, the dilution reagent can suppress hemolysis of red blood cell, and can promote staining of the blood cells with the fluorescent dye.

The highly-charged anion is added to the dilution reagent, in order to suppress non-specific staining of red blood cell with the fluorescent dye. The highly-charged anion is exemplified by sulfate ion, phosphate ion, carbonate ion, and polycarboxylate ion. Compounds capable of supplying these ions are exemplified by citric acid, sulfuric acid, phosphoric acid, EDTA, and alkali metal salts thereof. The highly-charged anion may be used singly or in combination of two or more kinds thereof.

The preservative contained in the dilution reagent is exemplified by sodium 2-pyridylthio-1-oxide and β-phenethyl alcohol.

The method for analyzing blood cells of this embodiment may employ a commercially available dilution reagent. The commercially available dilution reagent is exemplified by CELLPACK (registered trademark) DFL (Sysmex Corporation).

### (Staining Reagent and Fluorescent Dye)

Staining reagent used for preparing the measurement sample will be described. The fluorescent dye suitable for the staining reagent can be excited with light of the first wavelength, and can be bound to nucleic acid. This sort of fluorescent dye per se has been known, and is selectable from the group consisting of acridine-based compound, cyanine-based compound, and styryl-based compound. With such binding property to nucleic acid, the fluorescent dye makes it possible to distinguish nucleic acid-free cells such as mature red blood cell and platelet, from nucleic acid-containing cells such as reticulocyte and white blood cells.

The fluorescent dye that belongs to the acridine-based compound is exemplified by the one represented by the aforementioned formula (I). The "alkyl group" can be either linear or branched. The alkyl group having 1 to 18 carbon atoms, represented by R₁ to R₁₂ in formula (I), is exemplified by methyl, ethyl, propyl, isopropyl, t-butyl, n-butyl, isopentyl, neopentyl, t-pentyl, isohexyl, heptyl, octyl, decyl, dodecyl, tetradecyl, hexadecyl, and octadecyl. The alkyl group preferably has 1 to 10 carbon atoms, wherein the number of carbon atoms is more preferably 1 to 6, and is particularly 1 to 3. Preferred examples of the alkyl group include methyl, ethyl, propyl, isopropyl, t-butyl, n-butyl, isopentyl, neopentyl, t-pentyl, and isohexyl. The alkyl group represented by R₁₃ in formula (I) preferably has 1 to 3 carbon atoms.

The "halogen" is fluorine, chlorine, bromine, or iodine. "Haloalkyl group" is an alkyl group having at least one hydrogen substituted with halogen. In the haloalkyl group having two or more halogens, types of such halogens may be same or different. The "acylamino group" is a group (R-CO-NH-) having one hydrogen in the amino group substituted with an acyl group. The acylamino group is exemplified by formylamino, acetylamino, propionylamino, butyrylamino, benzoylamino, toluoylamino, naphthoylamino, nicotinoylamino, isonicotinoylamino, and furoylamino.

The substituent of the phenyl group is exemplified by -NH₂, halogen, -OH, - SH, -CN, -NO₂, -COOH, and alkyl group having 1 to 6 carbon atoms. The counter ion X⁻ in Formula (I) is exemplified by F⁻, Cl⁻, Br⁻, I⁻, 1/2SO₄²⁻, and NO₃⁻. With X⁻represented by Cl⁻, 1/2SO₄²⁻ or NO₃⁻, the fluorescent dye may contain an ion donating acid added thereto, such as HCl, H₂SO₄ or HNO₃. Lactic acid may alternatively be added to the fluorescent dye as the counter ion X⁻.

Preferred examples of the fluorescent dye that belongs to the acridine-based compound include 2,7,9-trimethylacridine-3,6-diamine, 3,6-diaminoacridine, 3,6-diamino-10-methylacridinium, 3,6-diamino-2,7,10-trimethylacridinium, 3,6-diamino-2,7-dimethylacridinium, N3,N3,N6,N6-trimethylacridine-3,6-diamine, 9-(4-aminophenyl)acridine-3-amine, N2,N2,N8,N8-tetramethylacridine-2,8-diamine, and 6,9-diamino-2-ethoxyacridine.

The fluorescent dye that belongs to the cyanine-based compound is exemplified by the one represented by the aforementioned formula (II). The alkyl group in formula (II) is the same as described regarding formula (I). The "alkenyl group" can be either linear or branched. The alkenyl group having 3 to 20 carbon atoms, represented by R₁ and R₂ in formula (II), is exemplified by allyl, propenyl, isopropenyl, butenyl, isobutenyl, pentenyl, isopentenyl, hexenyl, heptenyl, octenyl, decenyl, dodecenyl, tetradecenyl, hexadecenyl, octadecenyl, and icosenyl. The alkenyl group preferably has 3 to 14 carbon atoms, wherein the number of carbon atoms is more preferably 3 to 10, and is particularly 3 to 6.

The substituent on the phenyl group in formula (II) is the same as described regarding formula (I). The substituent on the benzyl group in formula (II) is exemplified by -CN, -COOH, -NH₂, -NO₂, -OH, -SH, alkyl group having 1 to 18 carbon atoms, alkoxy group having 1 to 6 carbon atoms, alkylamino group having 1 to 6 carbon atoms, acylamino group having 1 to 6 carbon atoms, halogen, and haloalkyl group having 1 to 6 carbon atoms. The counter ion Z⁻ in Formula (II) is exemplified by F⁻, Cl⁻, Br⁻, I⁻, CF₃SO₃⁻, BF₄⁻, and ClO₄⁻.

Preferred examples of the fluorescent dye that belongs to the cyanine-based compound represented by formula (II) include 3-methyl-2-[(3-methyl-2-benzoxazolinylidene)methyl]benzoxazolium, 3-ethyl-2-[(3-ethyl-2-benzothiazolinylidene)methyl]benzothiazolium, 3-ethyl-2-[(3-ethyl-6-methyl-2-benzothiazolinylidene)methyl]-6-methyl-benzothiazolium, 3-ethyl-2-[(3-ethyl-2-benzoxazolinylidene)methyl]benzoxazolium, 3-ethyl-2-[(3-ethyl-2-benzothiazolinylidene)methyl]benzoxazolium, 3-ethyl-6-methyl-2-[(3-ethyl-6-methyl-2-benzothiazolinylidene)methyl]benzothiazolium, 3-methyl-2-[(1,3,3-trimethyl-2-indolinylidene)methyl]benzothiazolium, and 1,3,3-trimethyl-2-[(3-methyl-2-benzoxazolinylidene)methyl]-3H-indolium.

The fluorescent dye that belongs to the acridine-based compound is exemplified by the one represented by the aforementioned formulae (III) and (IV). The substituent on the alkyl group and the phenyl group in formulae (III) and (IV) is the same as described regarding formula (I). Also the substituent on the alkenyl group and the benzyl group is the same as described regarding formula (II). In formula (IV), the heterocycle, formed by R₂ and/or R₃ together with the benzene ring to which N is bound, may be a five-membered ring or a six-membered ring. The heterocyclic ring may also have a substituent. The substituent is exemplified by alkyl group having 1 to 3 carbon atoms. The counter ion Z⁻ in formulae (III) and (IV) is exemplified by F⁻, Cl⁻, Br⁻, I⁻, CF₃SO₃⁻, BF₄⁻, and ClO₄⁻. The fluorescent dye that belongs to the styryl-based compound represented by formula (III) is exemplified by 3-ethyl-2-[(1-ethyl-2(1H)-quinolylidene)methyl]benzothiazolium. The fluorescent dye that belongs to the styryl-based compound represented by formula (IV) is typically exemplified by 2-[4-(dimethylamino)phenyl]-3,6-dimethyl-1,3-benzothiazol-3-ium.

The staining reagent preferably contains a solvent of the fluorescent dye. The solvent is not particularly limited as long as it can dissolve the fluorescent dye. The solvent is exemplified by water, organic solvent, and mixtures thereof. The organic solvent is exemplified by alcohol having 1 to 6 carbon atoms, ethylene glycol, diethylene glycol, polyethylene glycol, and dimethyl sulfoxide (DMSO). Concentration of the fluorescent dye in the staining reagent may be suitably determined, depending on types of the fluorescent dye. The concentration of the fluorescent dye is typically 100 ppm or higher, preferably 500 ppm or higher, and more preferably 1000 ppm or higher. The concentration of the fluorescent dye is typically 100000 ppm or lower, preferably 50000 ppm or lower, and more preferably 10000 ppm or lower.

Since the fluorescent dye itself can bind to the nucleic acid of the cell as described above, the method for analyzing blood cells of this embodiment no longer needs staining of the cells with use of an antibody labeled with the fluorescent dye. Hence in the method for analyzing blood cells of this embodiment, the fluorescent dye does not contain an antibody.

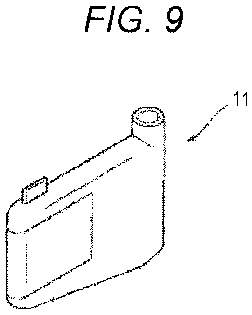

The staining reagent that contains the fluorescent dye may also be provided as a reagent for blood cell analysis, used for the method for analyzing blood cells of this embodiment. The reagent for blood cell analysis of this embodiment may typically have a form such that a solution of the fluorescent dye is filled in a container. Referring now to Fig. 9, reference sign 11 denotes a container, having the reagent for blood cell analysis of this embodiment filled therein. The container 11, when enclosed in a package box, may be typically accompanied by a package insert that describes composition of the reagent for blood cell analysis, directions for use, storage and so forth, and a cushion material for cushioning external impact, enclosed therein.

A further embodiment relates to use of the fluorescent dye, for manufacture of the reagent for blood cell analysis. Details of the fluorescent dye and the reagent for blood cell analysis are as described above.

### (Preparation of Measurement Sample)

The fluorescent dye is mixed with the specimen and the dilution reagent, so that the concentration (final concentration) in the measurement sample falls within a predetermined range. A preferred final concentration of the fluorescent dye in the measurement sample is 500 ppm or lower, which is more preferably 100 ppm or lower, and even more preferably 50 ppm or lower. A preferred final concentration of the fluorescent dye in the measurement sample is 0.01 ppm or higher, which is more preferably 0.1 ppm or higher, and even more preferably 1 ppm or higher.

The ratio of mixing of the dilution reagent, the staining reagent, and the specimen may be appropriately determined, according to the concentration of the fluorescent dye in the staining reagent, or types of the specimen. The ratio of mixing of the hemolytic reagent, the staining reagent, and the specimen is preferably, for example, 1000 : (0.1 or above) : (1 or above) on the volume basis. The ratio is more preferably 1000 : (0.5 or above) : (5 or above), and even more preferably 1000 : (1 or above) : (10 or above). The ratio of mixing of the dilution reagent, the staining reagent, and the specimen is preferably, for example, 1000 : (50 or below) : (100 or below) on the volume basis. The ratio is more preferably 1000 : (30 or below) : (50 or below), and even more preferably 1000 : (20 or below) : (25 or below). The ratio of mixing of the staining reagent and the specimen may be the same or different.

The method for analyzing blood cells of this embodiment enables detection of reticulocyte and platelet, without using a hemolysis reagent. Hence in the method for analyzing blood cells of this embodiment, the measurement sample does not contain any hemolysis reagent. That is, the method for analyzing blood cells of this embodiment does not include hemolyzing red blood cell, in the process of preparing the measurement sample. The hemolysis reagent herein is a reagent for lysing red blood cell in a specimen, upon mixed with the specimen. The hemolysis reagent itself has been known, and typically contains a surfactant whose concentration is enough for lysing red blood cell.

### (Analysis Process in First Embodiment)

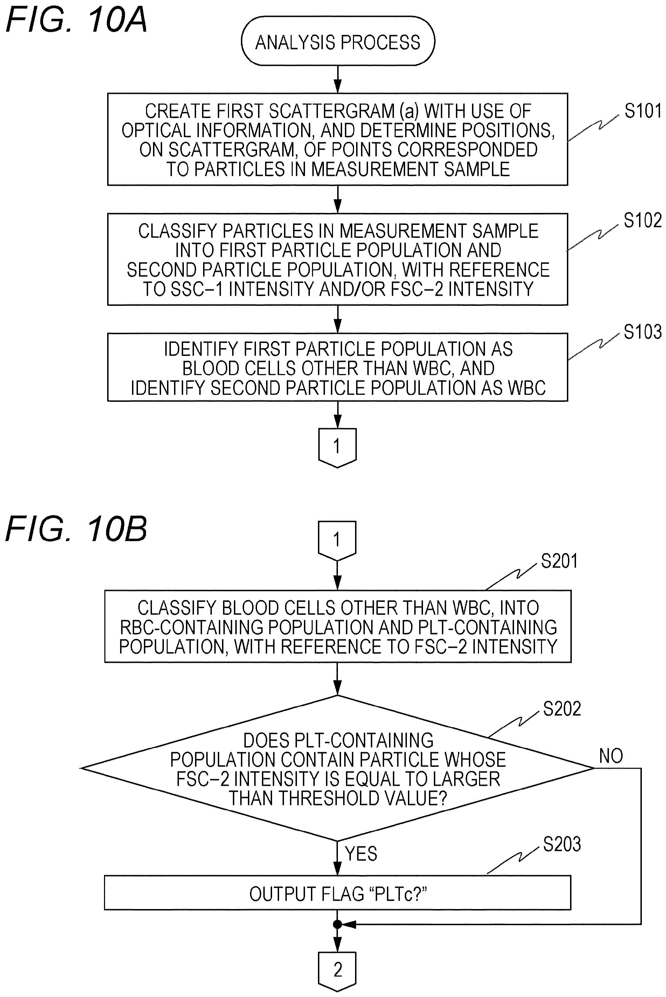

Exemplary analysis process in step S13 in Fig. 7 according to the first embodiment will be explained while referring to Figs. 10A to 17, without limiting the invention. In this specification, the mature red blood cell will be denoted as "mRBC", reticulocyte as "RET", platelet as "PLT", and white blood cell as "WBC".

In the analysis process in the first embodiment, first, the blood cells other than WBC are identified from among the particles in the measurement sample. In the method for analyzing blood cells of this embodiment, the measurement sample contains mRBC and RET, due to absence of a hemolysis reagent. The measurement sample also contains WBC and PLT. That is, the blood cells other than WBC means a particle population that contains mRBC, RET, and PLT. In the first embodiment, the blood cells other than WBC are identified with reference to the first scattered light information and the second scattered light information. This example employs the first side-scattered light intensity (also referred to as "SSC-1 intensity") as the first scattered light information, and the second forward-scattered light intensity (also referred to as "FSC-2 intensity") as the second scattered light information.

The blood cells other than WBC may be identified, typically by analysis on scattergrams. A scattergram for identifying the blood cells other than WBC with reference to the first and second scattered light information is also referred to as "first scattergram (a)". Referring now to Fig. 10 A, the analysis unit 300 in step S 101 creates a first scattergram (a). The first scattergram (a) is created typically with reference to the SSC-1 intensity and the FSC-2 intensity. For example, the analysis unit 300 creates a scattergram having the SSC-1 intensity and the FSC-2 intensity plotted on two coordinate axes, with reference to the acquired optical information. Positions of points corresponded to the individual particles in the measurement sample are then determined, on the thus created first scattergram (a). The abscissa and the ordinate of the first scattergram (a) may be expressed in either logarithmic or linear scale. The abscissa and the ordinate of the first scattergram (a) are preferably given in a logarithmic scale.

In step S102, the analysis unit 300 classifies the particles in the measurement sample into a first particle population and a second particle population, with reference to the SSC-1 intensity and/or the FSC-2 intensity. The second particle population demonstrates higher SSC-1 intensity and/or FSC-2 intensity than the first particle population. Now, the scattered light intensity of the particle populations on the scattergram is preferably compared, typically with use of statistical representative values of the scattered light intensity of the individual particle populations. The statistical representative value of the scattered light intensity of a certain particle population is a value that can be acquired from the scattered light intensity of the particles that constitute the particle population, and is exemplified by median value, mean value, and mode value. The median value is preferred.

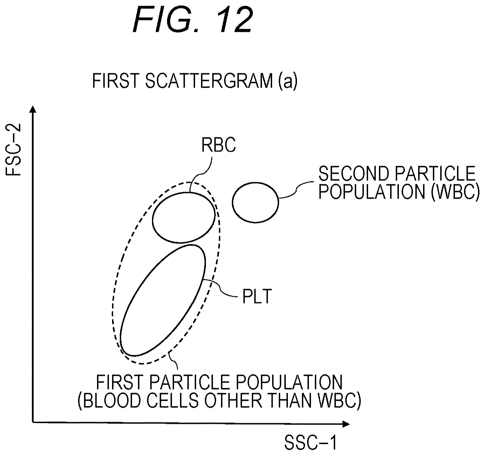

In the first scattergram (a), the first particle population appears in an area where the SSC-1 intensity and/or the FSC-2 intensity are low. Meanwhile, the second particle population appears in an area where the SSC-1 intensity and/or the FSC-2 intensity are high. In the example of Fig. 12, the first particle population appears in an area where the SSC-1 intensity is low, and the second particle population appears in an area where the SSC-1 intensity is high. The first particle population herein is a population constituted by particles that fall within an area surrounded by the broken line. More specifically, the first particle population encompasses a population that contains mRBC and RET (also referred to as "RBC-containing population"), and a PLT-containing population. That is, the first particle population contains the blood cells other than WBC. In a case where the specimen contains platelet aggregation, then the PLT-containing population also encompasses the platelet aggregation. The second particle population is a WBC population. Paragraphs below will explain how the particles in the measurement sample are classified into the first particle population and the second particle population on the first scattergram (a).

mRBC and RET (denoted as "RBC"), from among the blood cells other than WBC, can be distinguished from WBC, with reference to the SSC-1 intensity and/or the FSC-2 intensity. Now, hemoglobin contained in RBC has been known to absorb blue-violet light. RBC, when irradiated with the light of the first wavelength, which corresponds to blue-violet light, produces scattered light whose intensity is weaker than the scattered light expectedly produced from RBC irradiated with the light of the second wavelength, which corresponds to red light. Hence, the red light has been preferred for use in the prior hemanalysis. On the other hand, WBC demonstrates almost no difference in the intensity of the scattered light, either irradiated with the light of the first wavelength, or with the light of the second wavelength. The present inventors have arrived at an idea of discriminating WBC from RBC, making use of this finding. More specifically, the light of the first wavelength is irradiated to cause a difference in the first scattered light information, between WBC and RBC. For an exemplary case where the first scattered light information denotes the SSC-1 intensity, the SSC-1 intensity of RBC becomes lower due to absorption by hemoglobin. The SSC-1 intensity of the WBC, however, remains unaffected. The SSC-1 intensity of RBC therefore becomes lower than that of WBC. This enables discrimination between WBC and RBC. PLT and WBC, from among the blood cells other than WBC, are discriminable either by the FSC-2 intensity, or by the SSC-1 intensity. PLT is known to have the smallest size among the blood cells. The intensity of the forward-scattered light, generated upon irradiation of blood cells with light, corresponds to the size of the blood cells. PLT therefore produces the forward- scattered light whose intensity is lower than that of WBC. PLT also produces the side-scattered light whose intensity is lower than that of WBC, due to difference in internal structure. PLT and WBC are thus discriminable. Judging from the above, RBC and PLT, when collectively regarded as a single population (that is, the first particle population) on the first scattergram (a), will produce the SSC-1 intensity and the FSC-2 intensity, lower than those of the WBC population. Hence, the particles in the measurement sample can be classified into the first particle population that contains the blood cells other than WBC, and the second particle population that contains WBC, on the first scattergram (a).

Referring now to Fig. 10A, the analysis unit 300 in step S103 identifies the first particle population as the blood cells other than WBC. As described above, since the first particle population contains mRBC, RET, and PLT, so that the first particle population can be identified as the blood cells other than WBC. Meanwhile, the second particle population contains WBC, so that the second particle can be identified as WBC. Another possible method may be such as accumulating measurement data from a plurality of specimens to preliminarily determine the area on the first scattergram (a) where the first particle population will appear, and identifying the particle that appears in this area as the blood cells other than WBC.

As can be seen, the analysis process illustrated in Fig. 10A identifies the blood cells other than WBC, by classifying the particles in the measurement sample into WBC, and the blood cells other than WBC. In step S103, upon determination of the blood cells other than WBC, data regarding WBC is then deleted from data regarding particles in the measurement sample.

### (Detection of Platelet Aggregation)

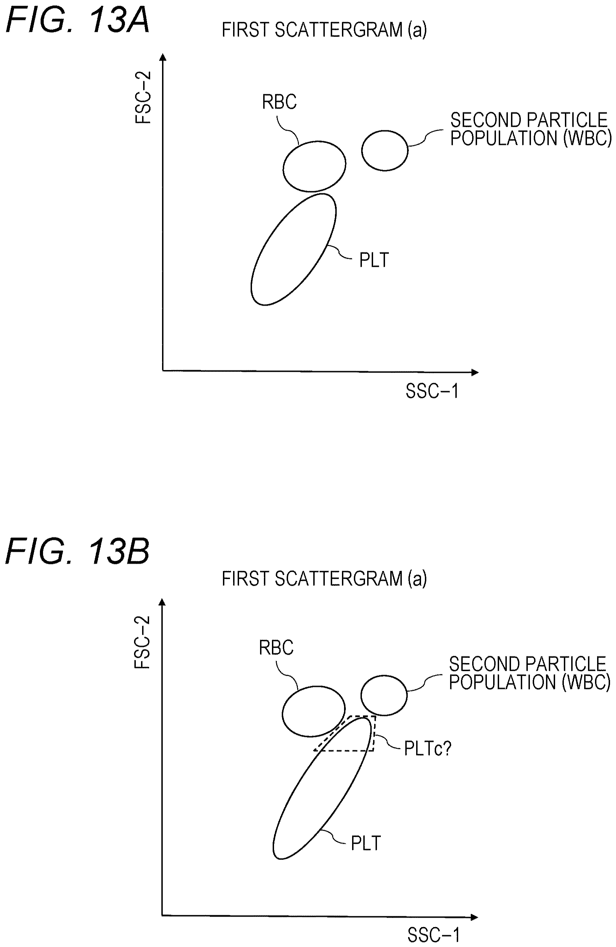

Referring now to Fig. 10B, the process advances to step S201. In this analysis process, information related to presence or absence of platelet aggregation is acquired. In step S201, the analysis unit 300 classifies the blood cells other than WBC, into the RBC-containing population, and the PLT-containing population, with reference to the FSC-2 intensity. Since PLT has a smaller size than mRBC or RET, so that the PLT-containing population demonstrates a lower FSC-2 intensity than the RBC-containing population. The cells other than WBC may, therefore, be classified into the RBC-containing population and the PLT-containing population, with reference to the FSC-2 intensity. Referring now to Fig. 13A, the RBC-containing population, the PLT-containing population, and the WBC population are distributed on the first scattergram (a). Another possible method may be such as accumulating measurement data of a plurality of specimens to preliminarily determine the areas on the first scattergram (a) where the RBC-containing population and the PLT-containing population will appear, and identifying the particles that appear in these areas as the RBC-containing population and the PLT-containing population.

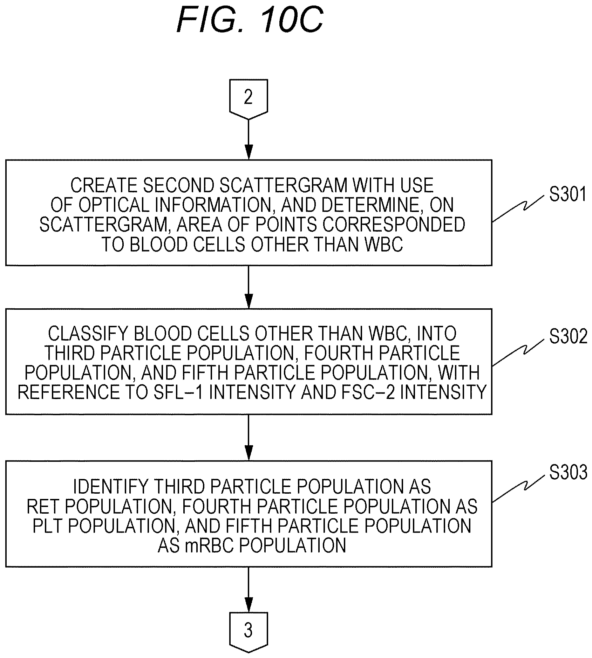

Referring now to Fig. 13B, illustrating a case where the specimen contains platelet aggregation, there are particles that distribute so as to extend from the PLT-containing population towards the WBC population, appeared on the first scattergram (a). In Fig. 13B, particles that appear in an area surrounded by the broken line indicate platelet aggregation. Platelet aggregation is an aggregate of a plurality of platelets, whose size is larger than that of normal platelet. The particles of platelet aggregation therefore demonstrate higher forward-scattered light intensity than platelet. Whether the PLT-containing population contains platelet aggregation or not can, therefore, be determined with reference to the FSC-2 intensity. Referring now to Fig. 10B, the analysis unit 300 in step S202 determines whether or not the PLT-containing population contains any particle whose FSC-2 intensity is equal to or above a threshold value. More specifically, the FSC-2 intensity of all particles constituting the PLT-containing population is compared with a threshold value that corresponds to the FSC-2 intensity. The particles whose FSC-2 intensity is equal to or above the threshold value are suspected of forming the platelet aggregation. That is, the PLT-containing population is suspected of containing the platelet aggregation. If step S202 judged there are particles whose FSC-2 intensity is equal to or above the threshold value, the process advances to step S203. Meanwhile, if step S202 judges there are no particle whose FSC-2 intensity is equal to or above the threshold value, the process advances to step S301 in Fig. 10C.

In a further embodiment, particles whose FSC-2 intensity is equal to or above the threshold value may be counted. If step S202 judges the count of the particles, demonstrating the FSC-2 intensity equal to or above the threshold value, is equal to or above a predetermined value, the process advances to step S203. Meanwhile, if the count of the particles, demonstrating the FSC-2 intensity equal to or above the threshold value, is smaller than the predetermined value, the process advances to step S301 in Fig. 10C. Now, the predetermined value is a value used for determining whether or not the particles, demonstrating the FSC-2 intensity equal to or above the threshold value, are platelet aggregation.

In step S203, the analysis unit 300 outputs information indicating that there is platelet aggregation. For example, a flag "PLTc?" may be output on the display 305. The process then advances to step S301 in Fig. 10C. Note that no-display of the flag on the display 305 can be information that indicates the absence of platelet aggregation.

### (Detection of Reticulocyte and Platelet)

After identifying the blood cells other than WBC, the process then advances to step S301 in Fig. 10C. In the analysis process, the blood cells other than WBC are classified into the RET population and the PLT population, with reference to the fluorescence information and the second scattered light information. This identifies RET and PLT. The blood cells other than WBC may alternatively be classified into the RET population, PLT population and an mRBC population. This identifies RET, PLT, and mRBC. Referring now to Fig. 10C, paragraphs below will explain a case of classifying the blood cells other than WBC, into the RET population, the PLT population, and the mRBC population.

The blood cells other than WBC may be classified, typically by analysis on scattergrams. A scattergram for classifying the blood cells other than WBC into the RET population and the PLT population, with reference to the fluorescence information and the second scattered light information, is also referred to as "second scattergram". This example employs the first side-fluorescence intensity (also referred to as "SFL-1 intensity") as the fluorescence information, and the FSC-2 intensity as the second scattered light information. The second scattergram is created with reference to the SFL-1 intensity and the FSC-2 intensity. Referring now to Fig. 10C, the analysis unit 300 in step S301 creates the second scattergram. For example, the analysis unit 300 creates a scattergram having the SFL-1 intensity and the FSC-2 intensity plotted on two coordinate axes, with reference to the acquired optical information. Positions of points corresponded to the blood cells other than WBC are then determined, on the thus created second scattergram. The abscissa and the ordinate of the second scattergram may be expressed in either logarithmic or linear scale. The second scattergram, with the abscissa and the ordinate expressed in logarithmic scales, is suitable for PLT analysis. Meanwhile, the second scattergram, with the abscissa and the ordinate expressed in linear scales, is suitable for RET and mRBC analyses.

In step S302, the analysis unit 300 classifies the blood cells other than WBC, into a third particle population, a fourth particle population, and a fifth particle population, with reference to the SFL-1 intensity and the FSC-2 intensity. The third particle population demonstrates higher SFL-1 intensity, than the fifth particle population. The fourth particle population demonstrates lower FSC-2 intensity, than the third particle population. Comparison of the scattered light intensity and the fluorescence intensity among the particle populations on the scattergrams is as described above.

Fig. 14A illustrates examples of the second scattergram (in logarithmic scale) and the second scattergram (in linear scale). There are the third particle population, the fourth particle population, and the fifth particle population appeared on the second scattergram. In the second scattergram (in logarithmic scale), the third and fifth particle populations appear in areas where the FSC-2 intensity is high, meanwhile the fourth particle population appears in an area where the FSC-2 intensity is low. In the second scattergram (in linear scale), the fourth particle population appears in an area where the FSC-2 intensity is low, meanwhile the third particle population appears in an area where the FSC-2 intensity is higher than that of the fourth particle population. In both scattergrams, the third particle population appears in the areas where the SFL-1 intensity is higher than that of the fifth particle population. As illustrated in Fig. 14A, the third particle population is the RET population, the fourth particle population is the PLT population, and the fifth particle population is the mRBC population. Paragraphs below will explain how the blood cells other than WBC are classified into the RET population, the mRBC population, and the PLT population, on the second scattergram.

As has been described previously, the PLT has the smallest size among the blood cells, and thus produces the forward-scattered light whose intensity is lower than that of RET. The PLT population therefore appears on the second scattergram, in the area where the FSC-2 intensity is lower than that of the RET population. RET and PLT are thus discriminable, with reference to the FSC-2 intensity. As has been described previously, RET contains a small amount of ribonucleic acid, meanwhile mRBC contains neither nucleus nor nucleic acid. The RET population therefore appears in the area where the fluorescence intensity is higher than that of the mRBC population. mRBC and RET are thus discriminable, with reference to the SFL-1 intensity. As can be seen, the second scattergram teaches that the blood cells other than WBC are classified into the third particle population that contains RET, the fourth particle population that contains PLT, and the fifth particle population that contains mRBC.

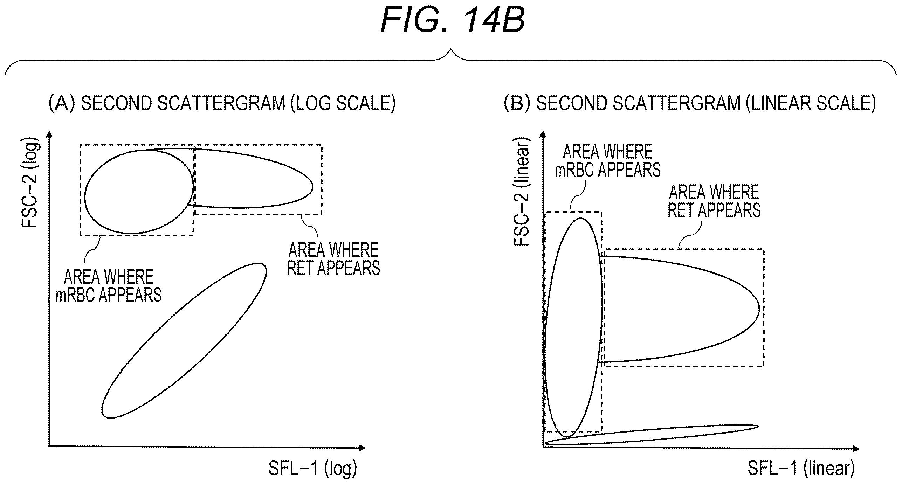

As described above, the third particle population contains RET, the fourth particle population contains PLT, and the fifth particle population contains mRBC. Referring now to Fig. 10C, the analysis unit 300 in step S303 identifies the third particle population as RET, the fourth particle population as PLT, and the fifth particle population as mRBC. RET matures to mRBC, and this makes it difficult to exactly define the boundary between the RET population and the mRBC population. It is therefore conceived, for example, to set a threshold value of the SFL-1 intensity at which the mRBC population and the RET population are dividable, typically with reference to a range of the SFL-1 intensity that can be usually demonstrated by mRBC, or an average level of the mRBC count. Such threshold value may be defined as a boundary between the mRBC population and the RET population on the second scattergram. The areas where the mRBC and RET individually appear may be set with reference to the threshold value and the FSC-2 intensity, typically as indicated by broken lines in Fig. 14B. Particles in the thus set area where mRBC appears can be identified as mRBC. Meanwhile, particles in the thus set area where RET appears can be identified as RET. The fourth particle population may be identified by the user as the PLT population on the second scattergram, if the third and fifth particle populations are clearly separated from the fourth particle population on the second scattergram. Alternatively, the areas where the third, fourth, and fifth particle populations will appear are preliminarily determined on the second scattergram, by accumulating measurement data from a plurality of specimens, and the particles that appeared in the individual areas may be identified as RET, PLT or mRBC. The thus identified RET, PLT, and mRBC may be optionally counted. The cells are counted with use of the analysis unit 300.

### (Analysis of Platelet, Analysis of Reticulocyte, and Classification of White Blood Cell)

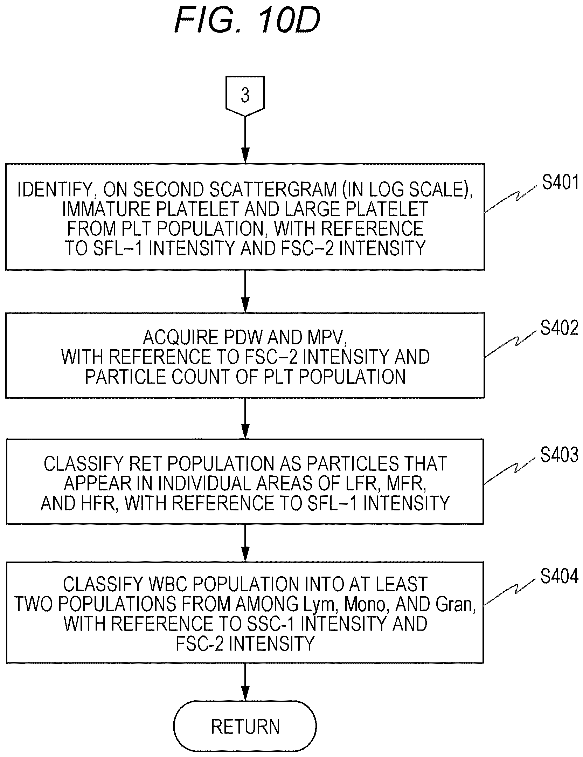

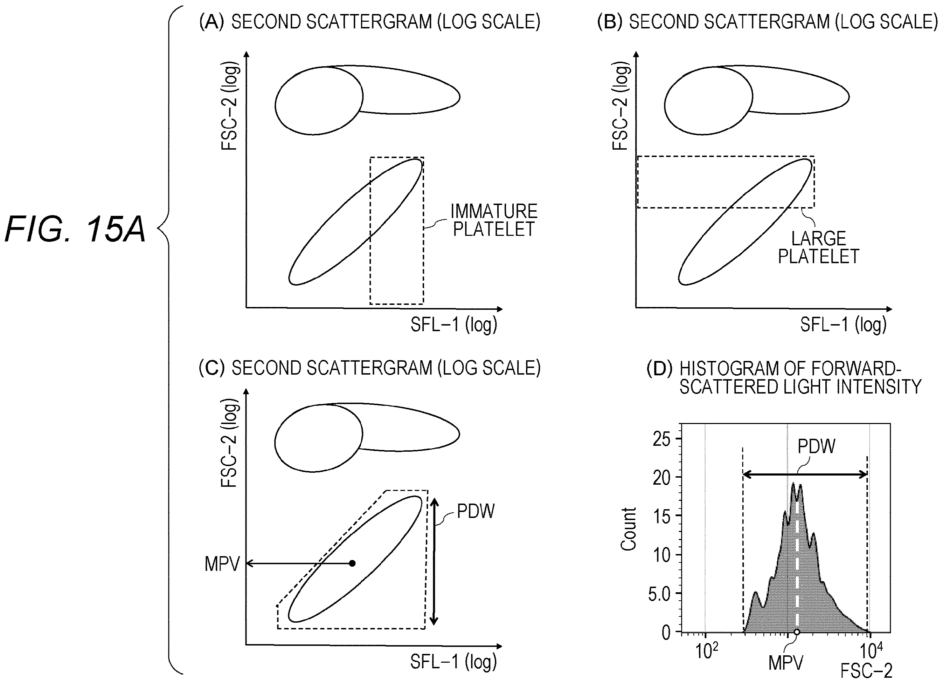

Referring now to Fig. 10D, the process advances to step S401. This analysis process further analyzes the PLT population and the RET population, and classifies the WBC population into subpopulations. In step S401, the analysis unit 300 identifies immature platelet and large platelet from the PLT population on the second scattergram (in logarithmic scale), with reference to the SFL-1 intensity and the FSC-2 intensity. As has been described previously, the mature platelet does not contain nucleic acid, meanwhile the immature platelet contains a small amount of ribonucleic acid. Hence, PLT that demonstrates high SFL-1 intensity may be identified as immature platelet, from among the PLT population on the second scattergram. For example, as illustrated in (A) in Fig. 15A, PLT that appears in an area where SFL-1 is high (the area surrounded by the broken line) on the second scattergram, may be identified as immature platelet. PLT other than immature platelet may be identified as mature platelet. Thus, the PLT population is classified into immature platelet and mature platelet. The identified immature platelet may be counted. Immature platelet fraction (IPF) may be estimated, if the platelet count has already been acquired by counting the PLT population. IPF represents a ratio of platelet count in an area with high fluorescence intensity (the area surrounded by a solid-lined square), to the platelet count. IPF has been known as an index of platelet productivity.

Since the large platelet has larger size than that of normal PLT, so that PLT that demonstrates high FSC-2 intensity, from among the PLT population on the second scattergram, may be identified as the large platelet. For example, as illustrated in the second scattergram named (B) in Fig. 15A, PLT that appears in an area where FSC-2 is high (the area surrounded by the broken line) may be identified as large platelet. PLT other than the large platelet may be identified as normal-sized PLT. Thus, the PLT population is classified into large platelet and normal-sized platelet. The identified large platelet may be counted. Percentage of large platelet (P-LCR) may be estimated, if the platelet count has already been acquired by counting the PLT population. P-LCR represents a ratio of platelet count in an area with high forward-scattered light intensity (the area surrounded by a broken-lined square), to the platelet count. P-LCR has been known as an index of platelet productivity and congenital diseases.

Referring now to Fig. 10D, the process advances to step S402. In step S402, the analysis unit 300 acquires the platelet distribution width (PDW) and the mean platelet volume (MPV), with reference to the FSC-2 intensity and the particle count of the PLT population. PDW represents a distribution of the forward-scattered light intensity of the PLT population, and gives an index that indicates variation in platelet size. MPV represents a mean value of the forward-scattered light intensity of the PLT population, and gives an index that indicates PLT size. As illustrated in (C) in Fig. 15A, PDW and MPV may be acquired by analyzing the PLT population on the second scattergram. PDW represents the length in the ordinate direction of the PLT population. MPV is a mean value estimated from the FSC-2 intensity of all particles that constitute the PLT population. MPV of the PLT population on the second scattergram may alternatively be determined, by creating a histogram with reference to FSC-2 intensity and the particle count, as illustrated in (D) in Fig. 15A. PDW is given by the length in the direction of abscissa of the histogram.

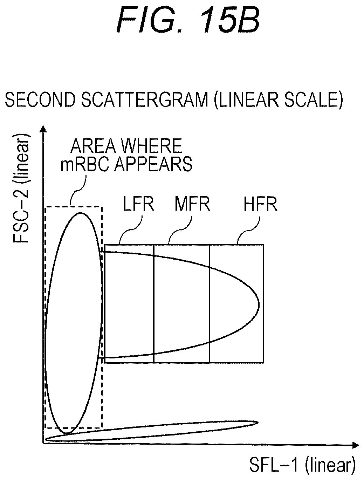

Referring now to Fig. 10D, the process advances to step S403. In step S403, the analysis unit 300 classifies the RET population, into particle that appears in the LFR area, particle that appears in the MFR area, and particle that appears in the HFR area, with reference to the SFL-1 intensity. It has been known that the younger the RET, the more abundant the nucleic acid (RNA) in the cell. As RET matures, the RNA level in the cell decreases. That is, the RNA level in RET indicates the degree of maturation of RET. Now, the SFL-1 intensity of the cell measured by FCM reflects the nucleic acid level in the cell. The degree of maturation of RET may therefore be analyzed with reference to the SFL-1 intensity. Referring to Fig. 15B, the area where RET appears on the second scattergram (in linear scale) can be divided into three areas of LFR, MFR, and HFR with reference to the SFL-1 intensity. RET that appears in the individual areas may be counted. RET that appears in the HFR area is most nucleic acid-rich, and therefore represents most immature RET.

Alternatively, RET that appears in the individual areas of LFR, MFR, and HFR may be analyzed to acquire a mean value of the fluorescence intensity (SFL-1 intensity) and a mean value of the forward-scattered light intensity (FSC-2 intensity). Yet alternatively, the particle in an area where mRBC appears (that is, mRBC) may be analyzed to acquire mean values of the SFL-1 intensity and the FSC-2 intensity. Therapeutic effect on red blood cell diseases (for example, pernicious anemia, iron deficiency anemia, etc.) can be monitored with reference to the mean values of the SFL-1 intensity and FSC-2 intensity of RET and mRBC that appear in the individual areas, according to the method described in Japanese Examined Patent No. 4474135 (the entire content of which will be incorporated herein by reference). More specifically, the mean values of the SFL-1 intensity and the FSC-2 intensity in the individual areas of LFR, MFR, and HFR are plotted on a graph having the SFL-1 intensity and the FSC-2 intensity plotted on two coordinate axes, and an approximate straight line is acquired by the least squares method. A slope value of the approximate straight line is then acquired. The slope value is periodically acquired from the day the treatment starts, so as to monitor any change in the value, whereby the effect of the treatment can be determined.

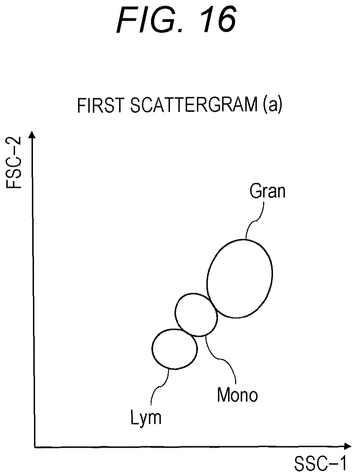

Referring now to Fig. 10D, the process advances to step S404. In this step, the WBC population identified in step S103 in Fig. 10A is classified into subpopulations. In step S404, the analysis unit 300 classifies the WBC population into subpopulations, with reference to the SSC-1 intensity and the FSC-2 intensity. The subpopulations of white blood cells are typically lymphocyte, monocyte, and granulocyte. The granulocyte is a generic term for neutrophil, eosinophil, and basophil. In step S404, an area where the WBC population appears on the first scattergram (a) is enlarged, whereby WBC is recognized to distribute while being divided into subpopulations, as illustrated in Fig. 16. In the drawings, "Gran" stands for granulocyte, "Mono" stands for monocyte, and "Lym" stands for lymphocyte. WBC in this drawing is classified into, but not limited to, three subpopulations. WBC can be classified into at least two populations, from among lymphocyte population, monocyte population, and granulocyte population. For example, WBC with some abnormality would occasionally decrease a part of the subpopulations, up to a degree that makes the classification difficult. WBC may be classified, for example, into two populations: the lymphocyte population or monocyte population, and granulocyte population.

WBC may also be classified into the subpopulations, typically as follows. First, areas where the individual subpopulations of WBC will appear on the first scattergram (a) are preliminarily determined, by accumulating measurement data from a plurality of specimens. The particle that appears in this area is then identified as the subpopulation of WBC. Cells contained in the individual subpopulations of WBC may be optionally counted. After completion of the classification of WBC, the analysis unit 300 finishes the analysis process. The process then advances to step S14 in Fig. 7.

### (Modified Example of Classification Process of White Blood Cell)

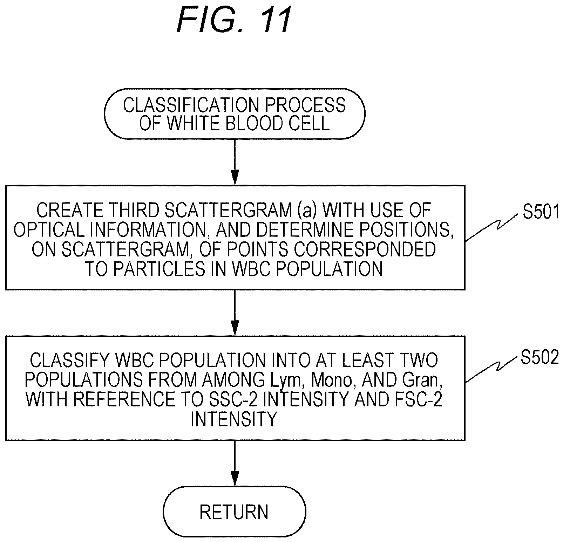

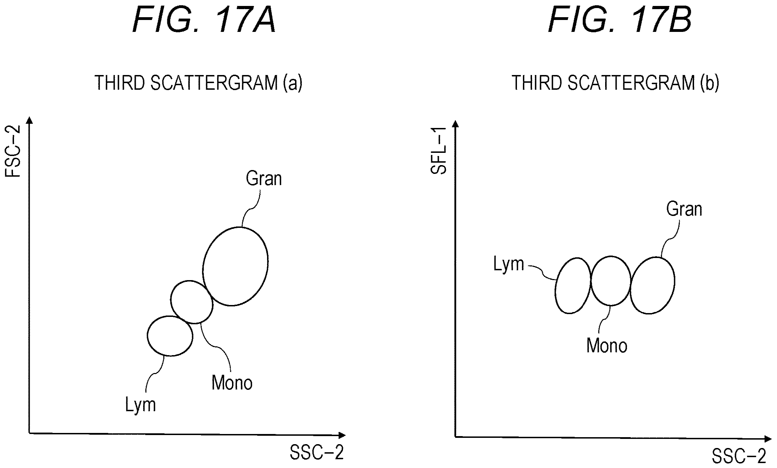

In step S404 in Fig. 10D, the WBC population was classified with reference to the first scattered light information and the second scattered light information. An exemplary analysis process for classifying the WBC population into subpopulations, with reference to the second scattered light information and the third scattered light information will be explained, while referring to Figs. 11 and 17. A scattergram for classifying the WBC population into subpopulations with reference to the second scattered light information and the third scattered light information is also referred to as "third scattergram (a)". This example employs the second side-scattered light intensity (also referred to as "SSC-2 intensity") as the third scattered light information. The third scattergram (a) is created typically with reference to the FSC-2 intensity and the SSC-2 intensity.

In step S501, the analysis unit 300 creates the third scattergram (a). For example, the analysis unit 300 creates a scattergram having the SSC-2 intensity and the FSC-2 intensity plotted on two coordinate axes, with reference to the acquired optical information. Positions of points corresponded to the individual particles in the WBC population are then determined, on the thus created third scattergram (a). The abscissa and the ordinate of the third scattergram (a) may be expressed in either logarithmic or linear scale. The abscissa and the ordinate of the third scattergram (a) are preferably given in a logarithmic scale. In step S502, the analysis unit 300 classifies the WBC population into lymphocyte, monocyte, and granulocyte, with reference to the SSC-2 intensity and the FSC-2 intensity. The granulocyte herein is a generic term for neutrophil, eosinophil, and basophil. On the third scattergram (a), WBC is recognized to distribute while being divided into subpopulations, as illustrated in (A) in Fig. 17. WBC in this drawing is classified into, but not limited to, three subpopulations. WBC can be classified into at least two populations, from among lymphocyte population, monocyte population, and granulocyte population. WBC may be classified, for example, into two populations: the lymphocyte population or monocyte population, and granulocyte population.

In a further embodiment, in the analysis process of Fig. 11, the WBC population may be classified with reference to fluorescence information and the third scattered light information. A scattergram for classifying the WBC population into subpopulations with reference to the fluorescence information and the third scattered light information is also referred to as "third scattergram (b)". For example, the third scattergram (b) is created typically with reference to the SFL-1 intensity and the SSC-2 intensity. Referring now to Fig. 11, the third scattergram (b) in step S501 may be created instead of the third scattergram (a). For example, the analysis unit 300 creates a scattergram with the SSC-2 intensity plotted on the abscissa, and with the SFL-1 intensity plotted on the ordinate, with reference to the acquired optical information. Positions of points corresponded to the individual particles in the WBC population are then determined, on the thus created third scattergram (b). The abscissa and the ordinate of the third scattergram (b) may be expressed in either logarithmic or linear scale. The abscissa and the ordinate of the third scattergram (b) are preferably given in a logarithmic scale. In step S502, the analysis unit 300 classifies the WBC population into lymphocyte, monocyte, and granulocyte, with reference to the SSC-2 intensity and the SFL-1 intensity. On the third scattergram (b) named (B) in Fig. 17, WBC is recognized to distribute while being divided into subpopulations. WBC in this drawing is classified into, but not limited to, three subpopulations. WBC population can be classified into at least two populations, from among lymphocyte population, monocyte population, and granulocyte population. WBC may be classified, for example, into two populations: the lymphocyte population or monocyte population, and granulocyte population.

WBC may also be classified into the subpopulations, typically as follows. First, areas where the individual subpopulations of WBC will appear on the third scattergrams (a) and (b) are preliminarily determined, by accumulating measurement data from a plurality of specimens. The particle that appears in this area is then identified as the subpopulation of WBC.

Cells contained in the individual subpopulations of WBC may be optionally counted. After completion of the classification of WBC, the analysis unit 300 finishes the analysis process. The process then advances to step S14 in Fig. 7.

### (Analysis Process of Second Embodiment)

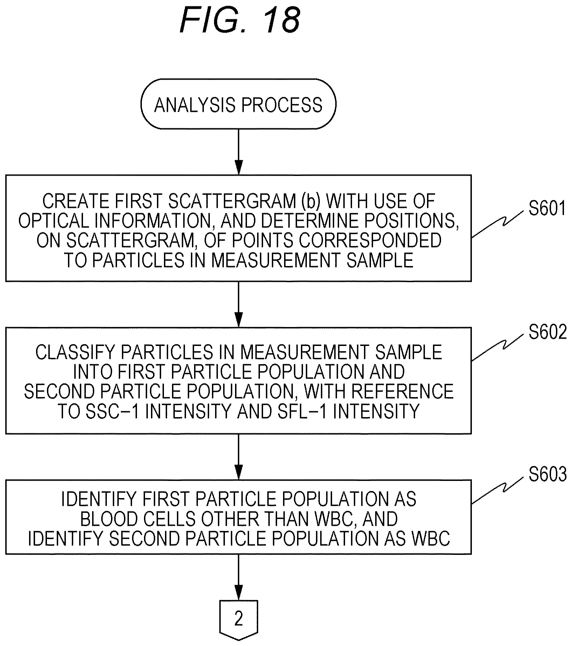

Exemplary analysis process in step S13 in Fig. 7 according to the second embodiment will be explained while referring to Figs. 18 to 20, without limiting the invention. In the second embodiment, the blood cells other than WBC are identified from among the particles in the measurement sample, with reference to the first scattered light information and the fluorescence information. This example employs the SSC-1 intensity as the first scattered light information, and the SFL-1 intensity as the fluorescence information. The blood cells other than WBC may be identified, typically by analysis on scattergrams. A scattergram for identifying the blood cells other than WBC with reference to the first scattered light information and the fluorescence information is also referred to as "first scattergram (b)". Referring now to Fig. 18, the analysis unit 300 in step S601 creates the first scattergram (b). The first scattergram (b) is created typically with reference to the SSC-1 intensity and the SFL-1 intensity. For example, the analysis unit 300 creates a scattergram having the SSC-1 intensity and the SFL-1 intensity plotted on two coordinate axes, with reference to the acquired optical information. Positions of points corresponded to the individual particles in the measurement sample are then determined, on the thus created first scattergram (b). The abscissa and the ordinate of the first scattergram (b) may be expressed in either logarithmic or linear scale. The abscissa and the ordinate of the first scattergram (b) are preferably given in a logarithmic scale.

In step S602, the analysis unit 300 classifies the particles in the measurement sample into a first particle population and a second particle population, with reference to the SSC-1 intensity and the SFL-1 intensity. The second particle population demonstrates higher SFL-1 intensity, than the first particle population. Now, the fluorescence intensity of the particle populations on the scattergram is preferably compared, typically with use of statistical representative values of the fluorescence intensity of the individual particle populations. The statistical representative value of the fluorescence intensity of a certain particle population is a value that can be acquired from the fluorescence intensity of the particles that constitute the particle population, and is exemplified by median value, mean value, and mode value. The median value is preferred. The statistical representative value of the fluorescence intensity of the particle population is calculated by the analysis unit 300.

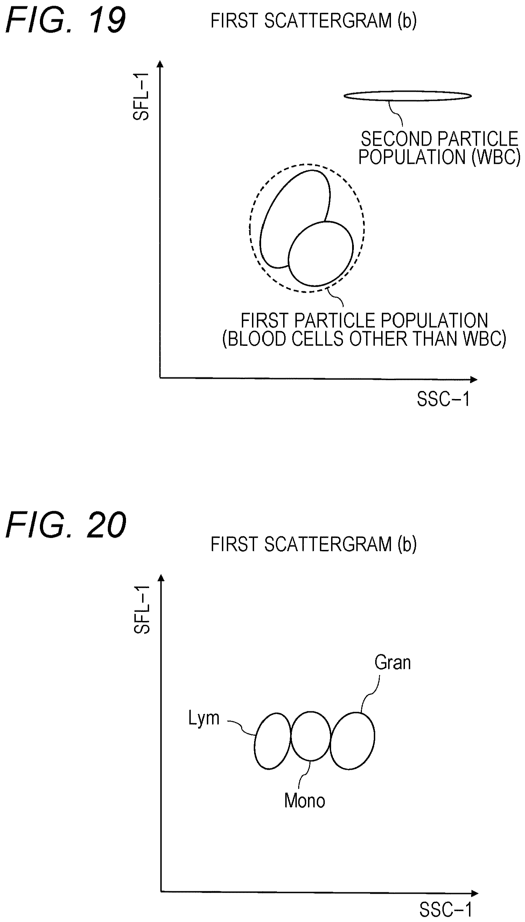

On the first scattergram (b), the first particle population appears in an area where the SSC-1 intensity and/or the SFL-1 intensity are low. Meanwhile, the second particle population appears in an area where the SSC-1 intensity and/or the SFL-1 intensity are high. In the example of Fig. 19, the first particle population appears in an area where the SSC-1 intensity and the SFL-1 intensity are low, meanwhile the second particle population appears in an area where the SSC-1 intensity and the SFL-1 intensity are high. The first particle population herein is a population constituted by particles that fall within an area surrounded by the broken line. More specifically, the first particle population contains mRBC, RET and PLT. That is, the first particle population contains the blood cells other than WBC. The second particle population is a WBC population. WBC, which is nucleic acid-rich, is more strongly stained with the aforementioned fluorescent dye. On the other hand, mRBC and PLT, which are free of nucleus or nucleic acid, are scarcely stained or weakly stained with the aforementioned fluorescent dye. RET contains ribonucleic acid, but in small amounts. RET does not have a nucleus. RET is therefore stained more weakly than WBC. As can be seen, there is a difference in fluorescence intensity between WBC, and the blood cells other than WBC. Hence, the particles in the measurement sample can be classified into the first particle population that contains the blood cells other than WBC, and the second particle population that contains WBC, on the first scattergram (b).

In step S603, the analysis unit 300 identifies the first particle population as the blood cells other than WBC. As described above, since the first particle population contains mRBC, RET, and PLT, so that the first particle population can be identified as the blood cells other than WBC. Meanwhile, the second particle population contains WBC, so that the second particle can be identified as WBC. Another possible method may be such as accumulating measurement data from a plurality of specimens to preliminarily determine the area on the first scattergram (b) where the first particle population will appear, and identifying the particle that appears in this area as the blood cells other than WBC.

As can be seen, the analysis process illustrated in Fig. 18 identifies the blood cells other than WBC, by classifying the particles in the measurement sample into WBC, and the blood cells other than WBC. In step S603, upon determination of the blood cells other than WBC, data regarding WBC is then deleted from data regarding particles in the measurement sample.

After identifying the blood cells other than WBC in step S603, the process then advances to step S301 in Fig. 10C. From among the blood cells other than WBC, then classified are RET, PLT, and mRBC, in the same manner as in the first embodiment. Details of the analysis process are same as those described previously in the first embodiment. Referring now to Fig. 10D, in the second embodiment, analysis of PLT, analysis of RET, and classification of WBC are implemented in the same manner as in the first embodiment. Details of the analysis process for PLT and RET are same as those described previously in the first embodiment. In step S404 in Fig. 10D, the WBC population identified in step S603 in Fig. 18 is classified into subpopulations, with reference to the SSC-1 intensity and the SFL-1 intensity. The subpopulations of white blood cell are typically lymphocyte, monocyte, and granulocyte. In step S404, an area where the WBC population appears on the first scattergram (b) is enlarged, whereby WBC is recognized to distribute while being divided into subpopulations, as illustrated in Fig. 20. WBC in this drawing is classified into, but not limited to, three subpopulations. WBC can be classified into at least two populations, from among lymphocyte population, monocyte population, and granulocyte population. For example, WBC with some abnormality would occasionally decrease a part of the subpopulations, up to a degree that makes the classification difficult. WBC may be classified, for example, into two populations: the lymphocyte population or monocyte population, and granulocyte population.

WBC may also be classified into the subpopulations, typically as follows. First, areas where the individual subpopulations of WBC will appear on the first scattergram (b) are preliminarily determined, by accumulating measurement data from a plurality of specimens. The particle that appears in this area is then identified as the subpopulation of WBC.

Cells contained in the individual subpopulations of WBC may be optionally counted. After completion of the classification of WBC, the analysis unit 300 finishes the analysis process. The process then advances to step S14 in Fig. 7.

Referring now to Fig. 7, upon completion of the aforementioned analysis process, the controller 301 in step S14 outputs analysis result on the display 305. The process thus finishes. The paragraphs above have individually described the processes for analyzing reticulocyte and platelet in the measurement sample, while referring to Figs. 10A to 20. The analysis process may, however, only be implemented in a part thereof, or may be implemented entirely.

An analysis result screen that is output on the display 305 may contain, for example, reticulocyte count, platelet count, IPF, P-LCR, PDW, MPV, flag of platelet aggregation, particle counts in the individual areas of LFR, MFR, and HFR, and the number of subpopulations of white blood cells (lymphocyte, monocyte, and granulocyte). Besides these information, the screen may contain a scattergram created with reference to the optical information. The screen may further contain a flag based on the analysis result. In an exemplary case where any particle suspected of platelet aggregation is identified, the screen may have output thereon information that indicates presence of platelet aggregation in the specimen. For example, a flag "PLTc?" may be output on the display 305.

As has been described, the method for analyzing blood cells of this embodiment can provide a medical worker such as doctor or medical technician, with analysis result of reticulocyte and platelet, such as information of reticulocyte count and platelet count in the specimen. The method can also provide analysis results of red blood cell and white blood cell. The medical worker can make decision from the acquired analysis results, on whether the specimen should further be inspected or not. All of the aforementioned preparation of the measurement sample and analysis of the specimen take place in vitro.

The present invention will be further detailed below, while referring to Examples, to which the present invention is however not limited.

### EXAMPLES

In the following Examples, the method for analyzing blood cells of this embodiment was implemented with use of the blood specimen, the dilution reagent, the staining reagent, and an analyzer below. Details will be described later in [Method of Measurement].

### [Blood Specimen]

Whole blood (peripheral blood) was collected from a plurality of subjects, with use of an EDTA-2K blood collection tube, and stored at room temperature (25 ± 2°C). The whole blood was fractionated from the blood collection tube at the time of use, to be used as the blood specimen. Details of the blood cell components contained in the blood specimen and/or the subject will be described later in the individual Examples.

### [Dilution Reagent]

CELLPACK (registered trademark) DFL (Sysmex Corporation) was used as the dilution reagent.

### [Staining reagent]

Various fluorescent dyes were dissolved in a special-grade ethylene glycol (1 L), to prepare the staining reagent. Types of the fluorescent dye used were described later in the individual Examples.

### [Analyzer]

Prototypes, modified from an automated hematology analyzer XN-20 (Sysmex Corporation) and a flow cytometer XF-1600 (Sysmex Corporation), were used as the analyzer. The individual prototypes were fabricated as follows. The original XN-20 has been equipped with a semiconductor laser light source that emits red (639 nm) light, a detector for red forward-scattered light, a detector for red side-scattered light, and a detector for fluorescence (660 nm or longer) excited by the red light. The XN-20 was further equipped with a semiconductor laser light source that emits blue-violet (405 nm) light, a detector for blue-violet side-scattered light, and a fluorescence detector that detects fluorescence (450 to 600 nm) excited by the blue-violet light. The XN-20 was further devised to analyze measurement data detected for the red and blue-violet scattered light information as well as the fluorescence information ascribed to the fluorescent dye, and to display desired scattergrams at will. The original XF-1600 has been equipped with three semiconductor laser light sources that emit blue-violet (405 nm) light, blue light (488 nm) and red (638 nm) light, detectors for scattered light of the individual colors, and detectors for fluorescence excited by lights of the individual colors. The XF-1600 was then modified, by detaching the semiconductor laser light source that emits blue (488 nm) light, to an FCM system having two semiconductor laser light sources. The individual prototypes have the hardware design illustrated in Figs. 1 to 3, and the FCM detector has a structure illustrated in Fig. 4.

### [Method of Measurement]

The preparation and measurement of the measurement sample were implemented according to manuals attached to XN-20 and XF-1600, except that CELLPACK DFL and the aforementioned staining reagent were used. The measurement sample was prepared by mixing 1000 µL of CELLPACK DFL, 5.0 µL of whole blood, and 20 µL of the staining reagent. The measurement sample was measured after incubation at 41°C for 30 seconds. The staining reagent was in 51.25× dilution in the measurement sample. The final concentration of the fluorescent dye in the measurement sample was described later in each Example.

### Example 1: Analysis of Blood Cells with Use of Acridine-Based Compound

A measurement sample was prepared with use of a staining reagent that contains an acridine-based compound as the fluorescent dye, and then analyzed with an analyzer to classify reticulocyte and platelet in the measurement sample.

### (1) Measurement Samples

The individual blood specimens were preliminarily subjected to classification and counting of red blood cell, reticulocyte, and platelet by a prior method, to acquire reticulocyte ratio (ratio of reticulocyte count to 1000 red blood cells, also referred to as "RET%" hereinafter). RET% is an index of anemia and hematopoietic abnormality, whose normal range is 0.4 to 2.0%. Classification and counting of red blood cell and reticulocyte by prior methods were implemented by measurement with use of an automated hematology analyzer XN-20 (Sysmex Corporation), and analysis with a RET channel. Classification and counting of platelet by a prior method were implemented by measurement with use of XN-20, and analysis with a PLT-F channel. In the classification and counting of platelets with use of XN-20, measured were particles six times as much as red blood cell or reticulocyte used in the classification and counting. From the blood specimens, those having RET% values of 0.21% and 5.56% (hereinafter, also referred to as "low-RET% specimen 1" and a "high-RET% specimen 1", respectively) were selected, and then used for the preparation of the measurement samples as described above. An ethylene glycol solution of 2,7,9-trimethylacridine-3,6-diamine was used as the staining reagent. The final concentration of the fluorescent dye in the measurement sample was 1.0 ppm.

### (2) Analysis

The individual measurement samples were measured with an analyzer (a prototype modified from XN-20), to acquire the first scattered light information, the second scattered light information, and the fluorescence information.

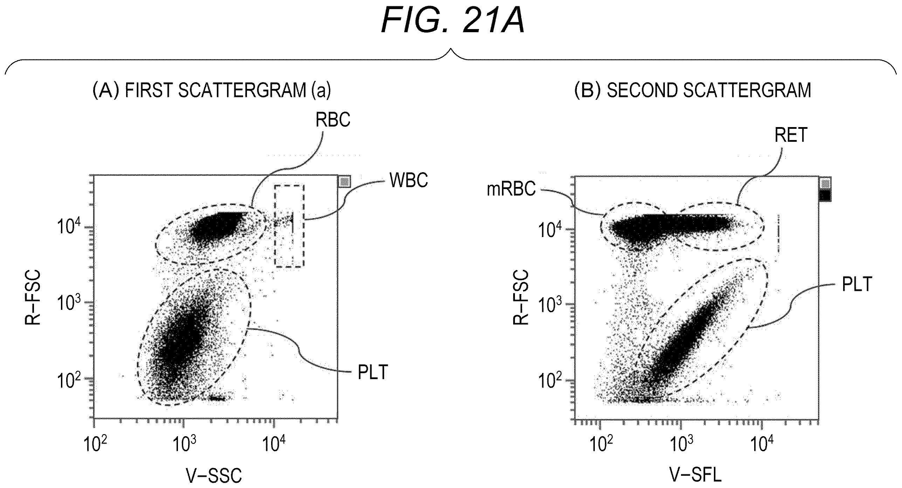

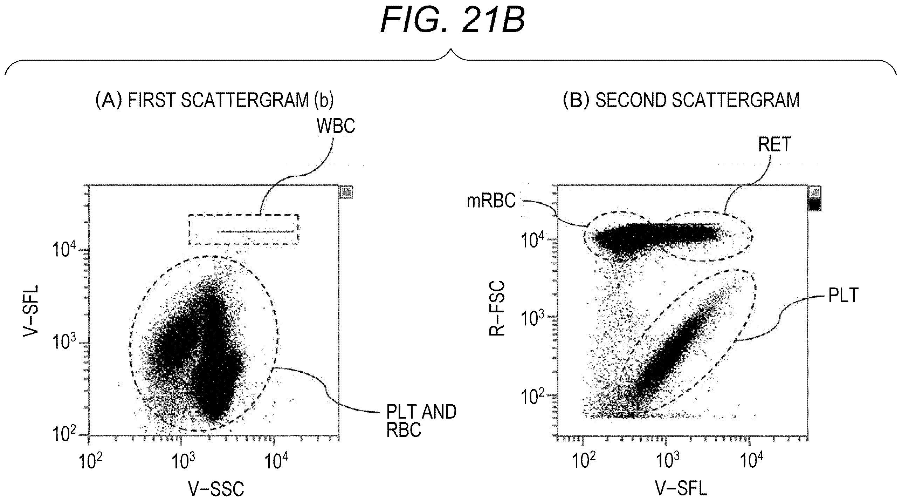

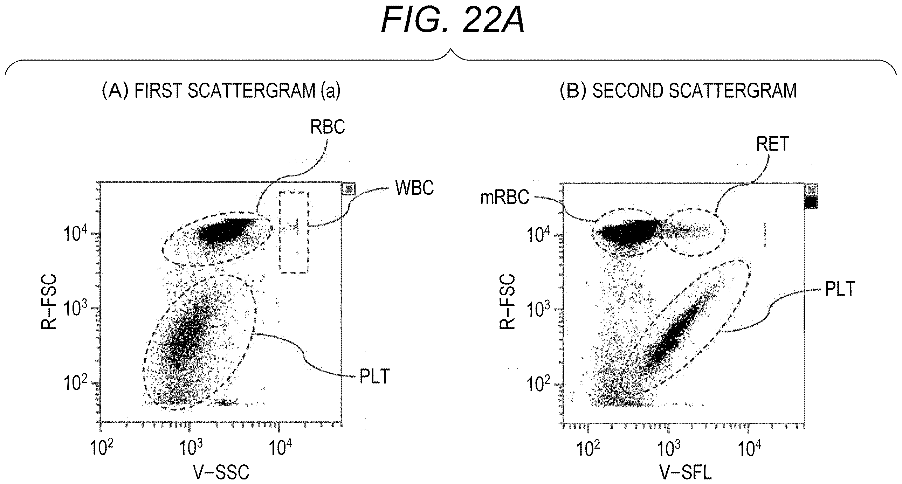

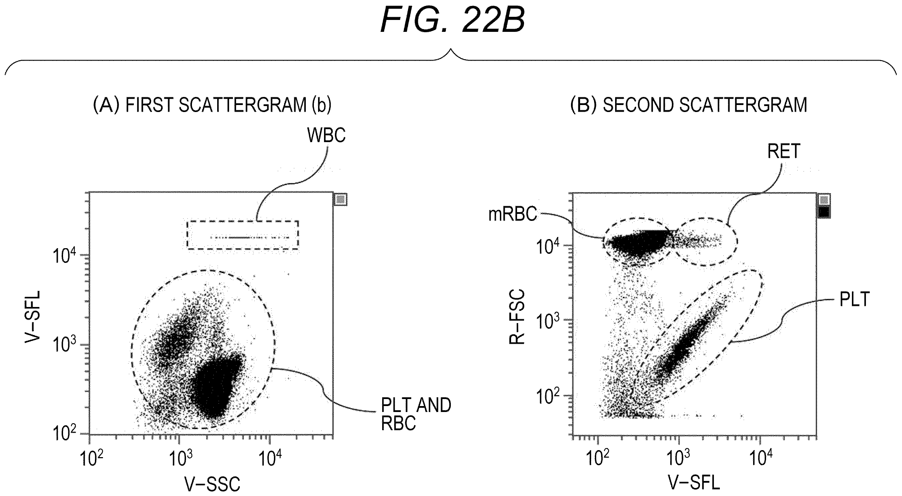

The first scattered light information corresponded to the side-scattered light intensity, obtained by irradiating the particles in the measurement sample with a blue-violet laser (also referred to as "side-scattered light intensity (blue-violet)" or "V-SSC", hereinafter). The second scattered light information corresponded to the forward-scattered light intensity, obtained by irradiating the particles in the measurement sample with a red laser (also referred to as "forward-scattered light intensity (red)" or "R-FSC", hereinafter). The fluorescence information corresponded to the fluorescence intensity, obtained by irradiating the particles in the measurement sample with a blue-violet laser (also referred to as "fluorescence intensity (blue-violet)" or "V-SFL", hereinafter). Scattergrams were created for the individual measurement samples, with the side-scattered light intensity (blue-violet) plotted on the abscissa, and with the forward-scattered light intensity (red) plotted on the ordinate (also referred to as "first scattergram (a)", hereinafter). Scattergrams were also created for the individual measurement samples, with the side-scattered light intensity (blue-violet) plotted on the abscissa, and with the fluorescence intensity (blue-violet) plotted on the ordinate (also referred to as "first scattergram (b)", hereinafter). The first scattergrams (a) and (b) of the high-RET% specimen 1 are illustrated in Figs. 21A and 21B, respectively. The first scattergrams (a) and (b) of the low-RET% specimen 1 are illustrated in Figs. 22A and 22B, respectively. Hereinafter in the drawings, "WBC" stands for white blood cell, "RBC" stands for red blood cell, "PLT" stands for platelet, "mRBC" stands for mature red blood cell, and "RET" stands for reticulocyte.

Referring now to the first scattergram (a) in Fig. 21A, the high-RET% specimen 1 caused white blood cell appeared in an area where V-SSC is high and R-FSC is high. The red blood cell population appeared in an area where V-SSC is lower than white blood cells, and R-FSC is high. The platelet population appeared in an area where V-SSC and R-FSC are lower than those of white blood cell. Platelet, having the size smaller than white blood cell and red blood cell, demonstrated lower R-FSC. The platelet population, therefore, appeared on the first scattergram (a), in an area where R-FSC is low. Red blood cell demonstrated low V-SSC, supposedly because hemoglobin in red blood cell absorbs the light of the first wavelength (blue-violet), as has been described previously. The red blood cell population on the first scattergram (a) is thus considered to appear in an area where V-SSC is lower than white blood cell. As can be seen, white blood cell was found to be distinguishable from the blood cells other than white blood cell, on the first scattergram (a). Blood cells other than white blood cell were sorted on the first scattergram (a). The sorted blood cells were plotted on a scattergram, with the fluorescence intensity (blue-violet) plotted on the abscissa, and with the forward-scattered light intensity (red) plotted on the ordinate (also referred to as "second scattergram", hereinafter). The abscissa and the ordinate of the second scattergram are in logarithmic scale.

Referring now to the second scattergram in Fig. 21A, the high-RET% specimen 1 caused white blood cell appeared in an area where V-SFL is low and R-FSC is high. The reticulocyte population appeared in an area where V-SFL is higher than that of the mature red blood cell population. The platelet population appeared in an area where R-FSC is lower than that of reticulocyte population. As can be seen, sorting of the blood cells other than white blood cell successfully classified mature red blood cell, reticulocyte, and platelet, while excluding influence of white blood cell. Referring now to the first scattergram (a) and the second scattergram in Fig. 22A, also the low-RET% specimen 1 gave results similar to those of the high-RET% specimen 1.

Referring now to the first scattergram (b) in Fig. 21B, the high-RET% specimen 1 caused white blood cell appeared in an area where V-SFL is high. A population that contains platelet and red blood cell appeared in an area where V-SFL is lower than that of white blood cell. Mature red blood cell and platelet are non-nucleated cells, and reticulocyte contains a small amount of RNA. In contrast, white blood cell has a nucleus. White blood cell therefore demonstrated V-SFL higher than that of the platelet population and the red blood cell population. White blood cell was found to be distinguishable from the blood cells other than white blood cell, also on the first scattergram (b), similarly to as on the first scattergram (a). With reference to the second scattergram in Fig. 21B, the blood cells other than white blood cell were classified into mature red blood cell, reticulocyte, and platelet. As can be seen, sorting of the blood cells other than white blood cell successfully classified platelet, mature red blood cell, and reticulocyte, while excluding influence of white blood cell. Referring now to the first scattergram (b) and the second scattergram in Fig. 22B, also the low-RET% specimen 1 gave similar result.

It was suggested that the influence of white blood cell can be reduced in the measurement of reticulocyte and platelet, by excluding white blood cell and classifying reticulocyte and platelet as described above. This example also successfully classified reticulocyte and platelet only with a one-time measurement, unlike the prior method that needed the measurement twice.

### Example 2: Analysis of Blood Cells with Use of Cyanine-Based Compound

Measurement samples were prepared with use of a staining reagent that contains a fluorescent dye different from that in Example 1, to classify reticulocyte and platelet in the measurement sample with use of an analyzer.

### (1) Measurement Samples

From the blood specimens, those having RET% values of 0.61% and 4,33% (hereinafter, also referred to as "low-RET% specimen 2" and a "high-RET% specimen 2", respectively) were selected, followed by preparation of the measurement samples as described above. An ethylene glycol solution of 3-ethyl-2- [(3-ethyl-2-benzothiazolinylidene)methyl]benzothiazolium was used as the staining reagent. The final concentration of the fluorescent dye in the measurement sample was 5.0 ppm.

### (2) Analysis

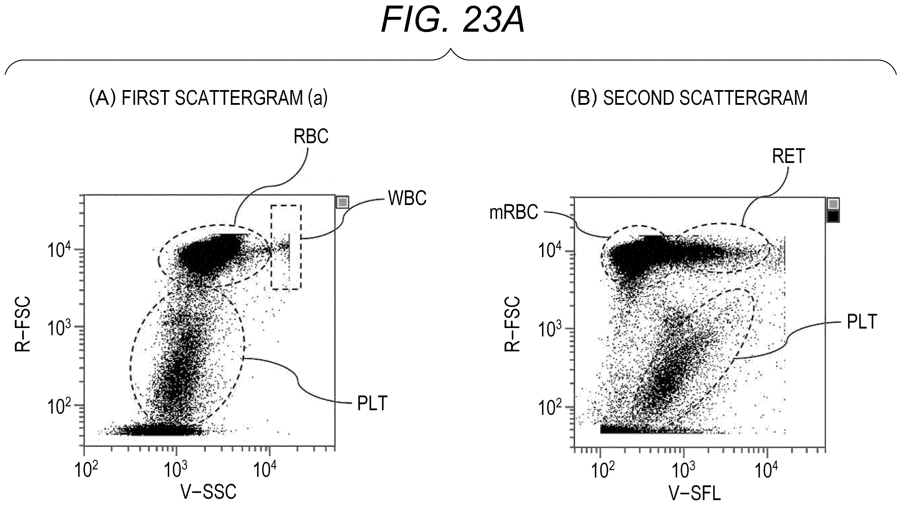

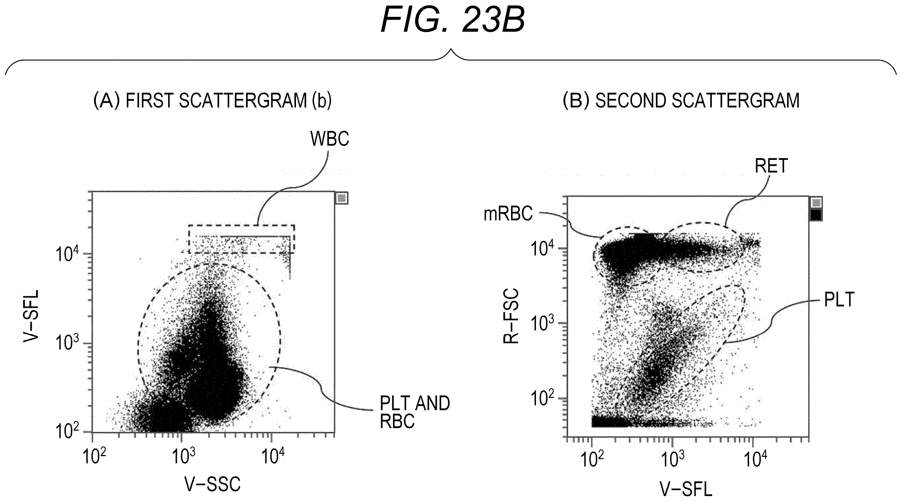

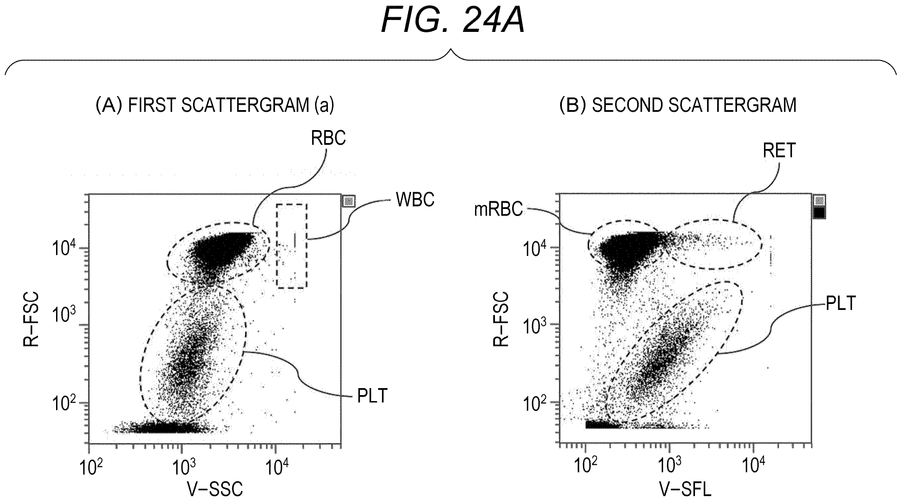

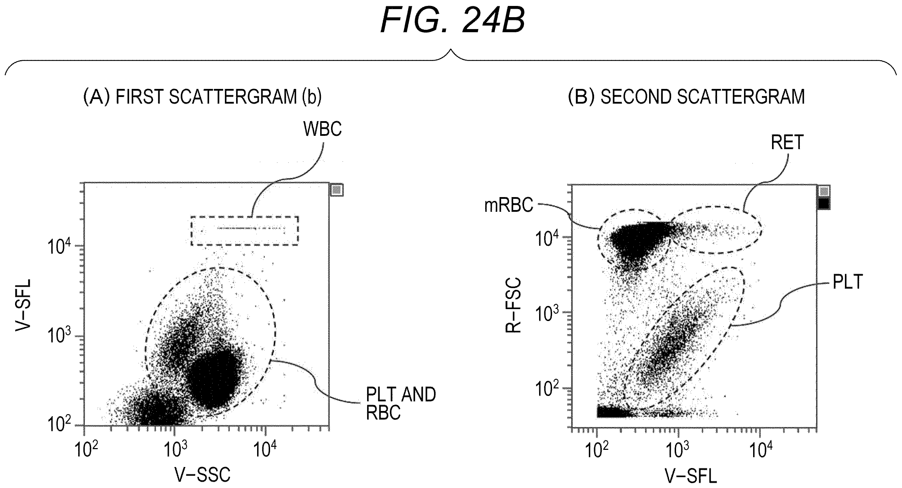

Similarly to as in Example 1, the individual measurement samples were measured with an analyzer (a prototype modified from XN-20), to acquire the first scattered light information, the second scattered light information, and the fluorescence information. The individual information were the same as those in Example 1. The first scattergrams (a) and (b) of the individual measurement samples were prepared, in the same manner as in Example 1. The first scattergrams (a) and (b) of the high-RET% specimen 2 are illustrated in Figs. 23A and 23B, respectively. The first scattergrams (a) and (b) of the low-RET% specimen 2 are illustrated in Figs. 24A and 24B, respectively.

Referring now to the first scattergram (a) in Fig. 23A, the high-RET% specimen 2 caused white blood cell appeared in an area where V-SSC is high and R-FSC is high. The red blood cell population appeared in an area where V-SSC is lower than that of white blood cell, and R-FSC is high. The platelet population appeared in an area where V-SSC and R-FSC are lower than those of white blood cell. As can be seen, white blood cell was found to be distinguishable from the blood cells other than white blood cell, on the first scattergram (a). The blood cells other than white blood cell were sorted on the first scattergram (a), and these blood cells were plotted on the second scattergram (in logarithmic scale). As can be seen in the second scattergram in Fig. 23A, sorting of the blood cells other than white blood cell successfully classified platelet, mature red blood cell, and reticulocyte, while excluding influence of white blood cell. Referring now to Fig. 24A, also the low-RET% specimen 2 gave similar results. Referring now to the first scattergram (b) in Fig. 23B, the high-RET% specimen 2 caused white blood cell appeared in an area where V-SFL is high. A population that contains platelet and red blood cell appeared in an area where V-SFL is lower than that of white blood cell. White blood cell was found to be distinguishable from the blood cells other than white blood cell, also on the first scattergram (b), similarly to as on the first scattergram (a). Sorting of the blood cells other than white blood cell on the first scattergram (b) successfully classified platelet, mature red blood cell, and reticulocyte, on the second scattergram, while excluding influence of white blood cell. Referring now to Fig. 24B, also the low-RET% specimen 2 gave similar results. These results taught that also use of the cyanine-based compound as the fluorescent dye successfully classified platelet and reticulocyte, similarly to as in Example 1 that used the acridine-based compound.

### Example 3: Analysis of Blood Cells with Use of Various Fluorescent Dyes

Measurement samples were prepared with use of staining reagents that contain fluorescent dyes different from those in Examples 1 and 2, to classify reticulocyte and platelet in the measurement sample with use of an analyzer. Comparative analyses were also implemented with use of staining reagents that individually contain 2,7,9-trimethylacridine-3,6-diamine, or 3-ethyl-2-[(3-ethyl-2-benzothiazolinylidene)methyl]benzothiazolium.

### (1) Measurement Samples

The individual blood specimens were preliminarily measured with XN-20 (Sysmex Corporation) and analyzed in the PLT-F channel, to acquire the platelet counts. Blood specimens having a platelet count of 50 × 10³/µL or lower were selected. From the selected blood specimens, selected were two blood specimens whose scattergrams in the PLT-F channel were found to contain debris populations that are different from the platelet population (hereinafter referred to as "low-PLT specimen 1" and a "low-PLT specimen 2", respectively). From these blood specimens, the measurement samples were prepared as described previously. The fluorescent dyes that belong to the acridine-based compound used herein were 2,7,9-trimethylacridine-3,6-diamine, 3,6-diamino -2,7,10-trimethylacridinium, 3,6-diamino-2,7-dimethylacridinium, 3,6-diaminoacridine, and 3,6-diamino-10-methylacridinium. The fluorescent dyes that belong to the cyanine-based compound used herein were 3-ethyl-2-[(3-ethyl-2-benzothiazolinylidene)methyl] benzothiazolium, 3-ethyl-2-[(3-ethyl-2-benzothiazolinylidene)methyl]benzoxazolium, 3-ethyl-6-methyl-2-[(3-ethyl-6-methyl-2-benzothiazolinylidene)methyl]benzothiazolium, 1,3,3-trimethyl-2-[(3-methyl-2-benzoxazolinylidene)methyl]-3H-indolium, and 3-ethyl-2-[(3-ethyl-6-methyl-2-benzothiazolinylidene)methyl]-6-methyl-benzothiazolium. Ethylene glycol solutions of the individual fluorescent dyes were used as the staining reagent. In the measurement sample, the final concentration of the fluorescent dye of the acridine-based compound was 1.0 ppm, and the final concentration of the fluorescent dye of the cyanine-based compound was 5.0 ppm.

### (2) Analysis

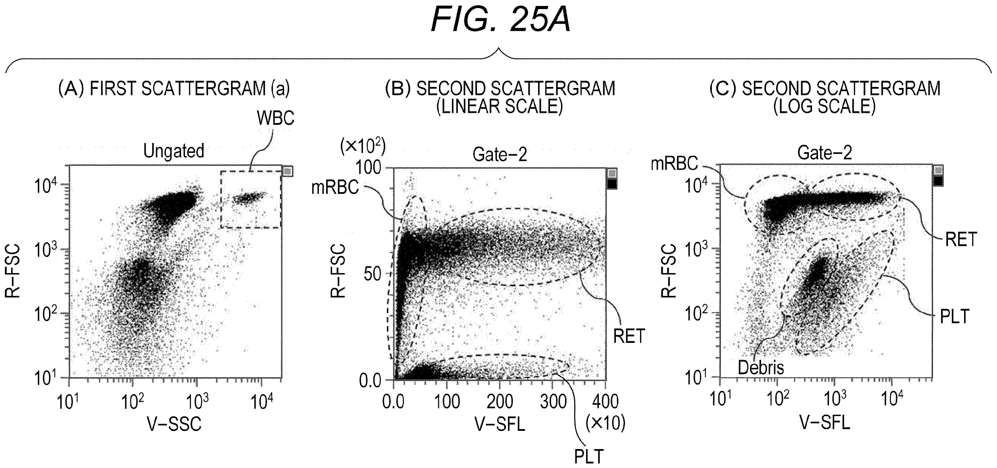

The individual measurement samples were measured with an analyzer (a prototype modified from XN-20), to acquire the first scattered light information, the second scattered light information, and the fluorescence information. The individual information were the same as those in Example 1. A first scattergram (a) was prepared for the measurement samples prepared with use of 3-ethyl-2-[(3-ethyl-2-benzothiazolinylidene)methyl] benzothiazolium, 3-ethyl-2-[(3-ethyl-2-benzothiazolinylidene)methyl]benzoxazolium, 3-ethyl-6-methyl-2-[(3-ethyl-6-methyl-2-benzothiazolinylidene)methyl]benzothiazolium, 3,6-diaminoacridine, or 1,3,3-trimethyl-2-[(3-methyl-2-benzoxazolinylidene)methyl]-3H-indolium. A first scattergram (b) was prepared for the measurement samples prepared with used of 2,7,9-trimethylacridine-3,6-diamine, 3,6-diamino-2,7,10-trimethylacridinium, 3,6-diamino-2,7-dimethylacridinium, 3,6-diamino-10-methylacridinium, or 3-ethyl-2-[(3-ethyl-6-methyl-2-benzothiazolinylidene)methyl]-6-methyl-benzothiazolium. The blood cells other than white blood cell were sorted on the first scattergrams (a) and (b), and were plotted on the second scattergrams. The second scattergrams were created in two ways, in linear scale and in logarithmic scale. These scattergrams are illustrated in Figs. 25A, 25B, 26A, 26B, 27A and 27B.

Fig. 25A contains examples of the first scattergram (a) and the second scattergram derived therefrom, illustrating analysis results of the measurement sample prepared from the low-PLT specimen 1 with use of 3-ethyl-2-[(3-ethyl-2-benzothiazolinylidene)methyl]benzothiazolium. Referring now to the first scattergram (a) in Fig. 25A, the white blood cell population appeared in an area where V-SSC is high and R-FSC is high. Referring now to the second scattergram (linear scale) in Fig. 25A, the mature red blood cell population appeared in an area where V-SFL is low, the platelet population appeared in an area where R-FSC is low, and the reticulocyte population appeared in an area where R-FSC is high. The population of debris was found to overlap with the platelet population. Referring now to the second scattergram (in logarithmic scale) in Fig. 25A, the mature red blood cell population appeared in an area where R-FSC is high and V-SFL is low. The reticulocyte population appeared in an area where R-FSC is equivalent to that of the mature red blood cell population, and V-SFL is higher than that of the mature red blood cell population. The platelet population and the debris population were distinguishable, although both appeared in an area where R-FSC is lower than that of mature red blood cell and reticulocyte. The second scattergram in logarithmic scale was thus proved to be suitable for classification and counting of platelet, and for discrimination between platelet and debris.

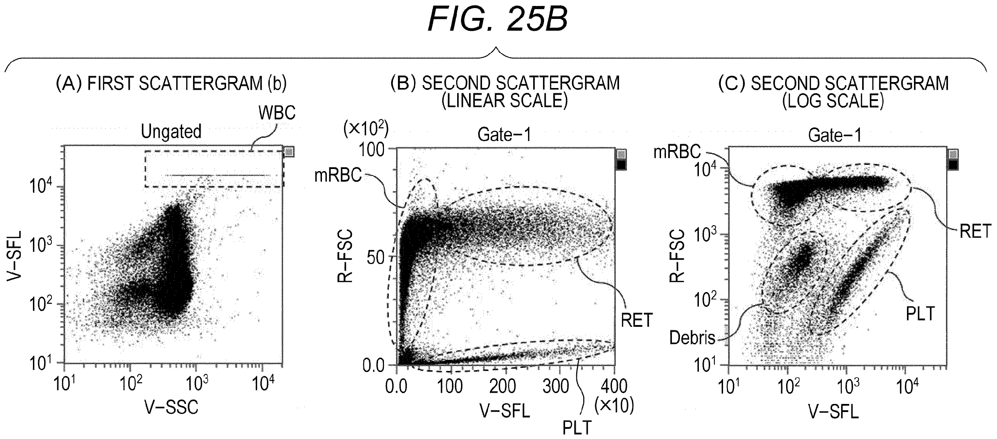

Fig. 25B contains the first scattergram (b) and the second scattergram derived therefrom, illustrating analysis results of the measurement sample prepared from the low-PLT specimen 1 with use of 2,7,9-trimethylacridine-3,6-diamine. Referring now to the first scattergram (b) in Fig. 25B, the white blood cell population appeared in an area where V-SFL is high. In the second scattergram (in linear scale) in Fig. 25B, there appeared the individual populations of mature red blood cell, reticulocyte, and platelet, similarly to as in the second scattergram (in linear scale) in Fig. 25A. In the second scattergram (in logarithmic scale) in Fig. 25B, there appeared the individual populations of mature red blood cell, reticulocyte, platelet, and debris. The platelet population and the debris population were found apart from each other, thus enabling discrimination between both populations.

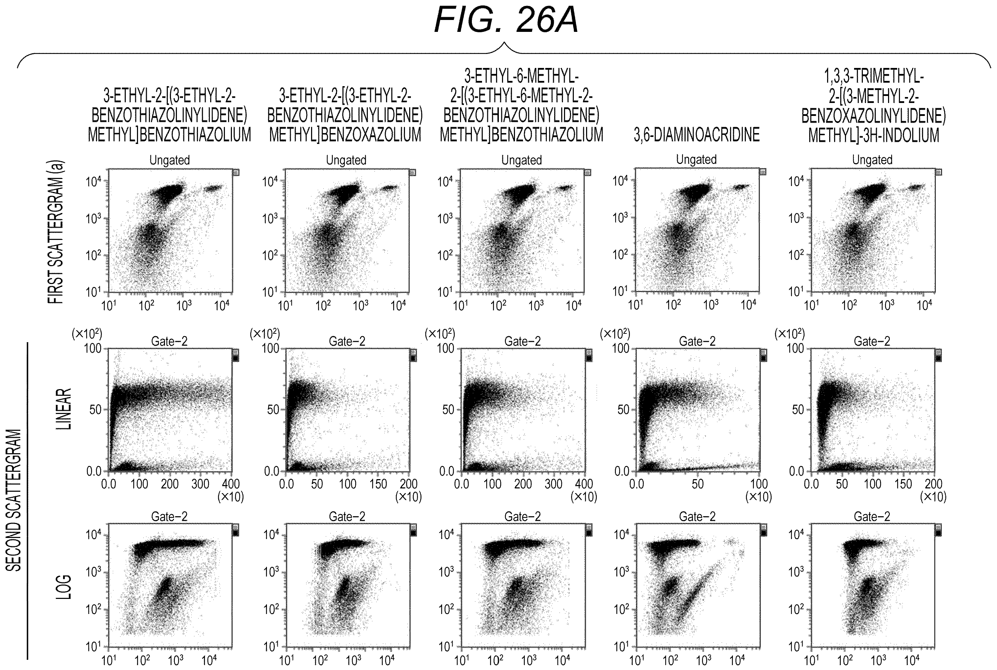

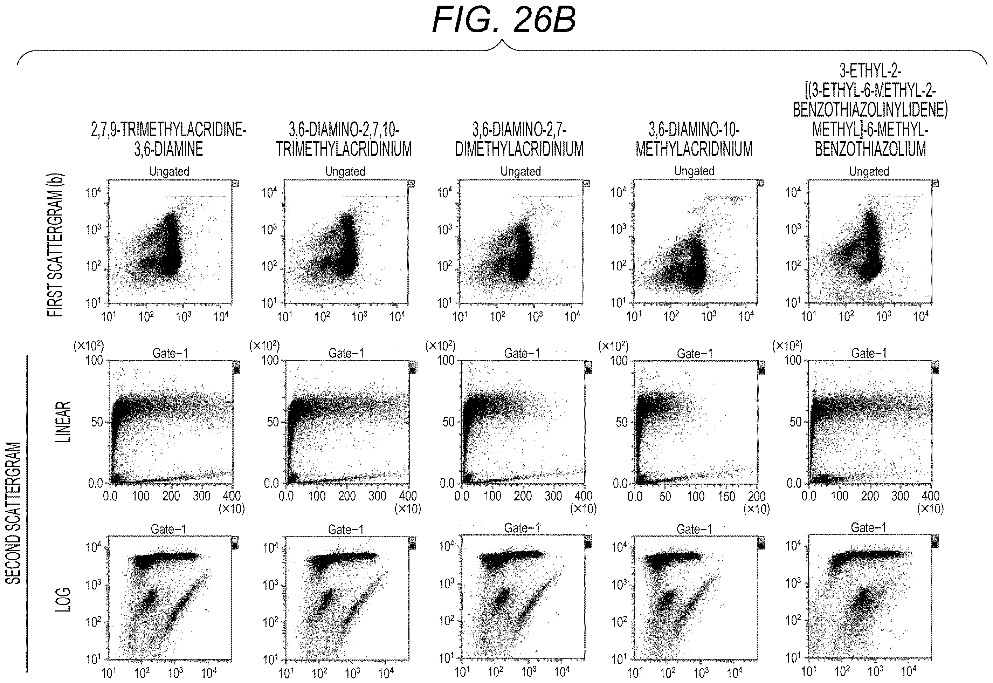

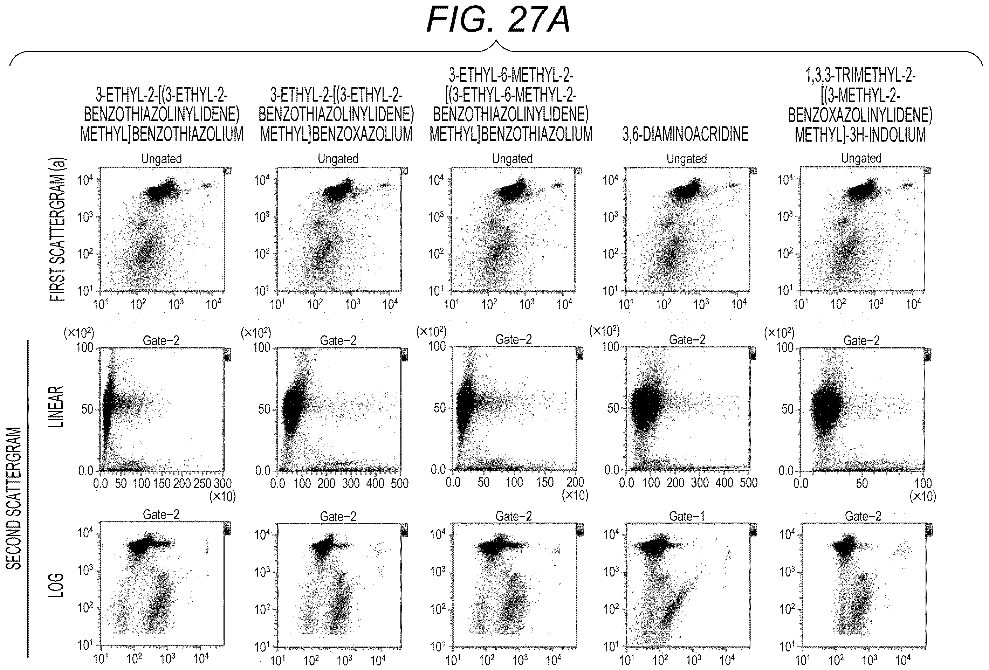

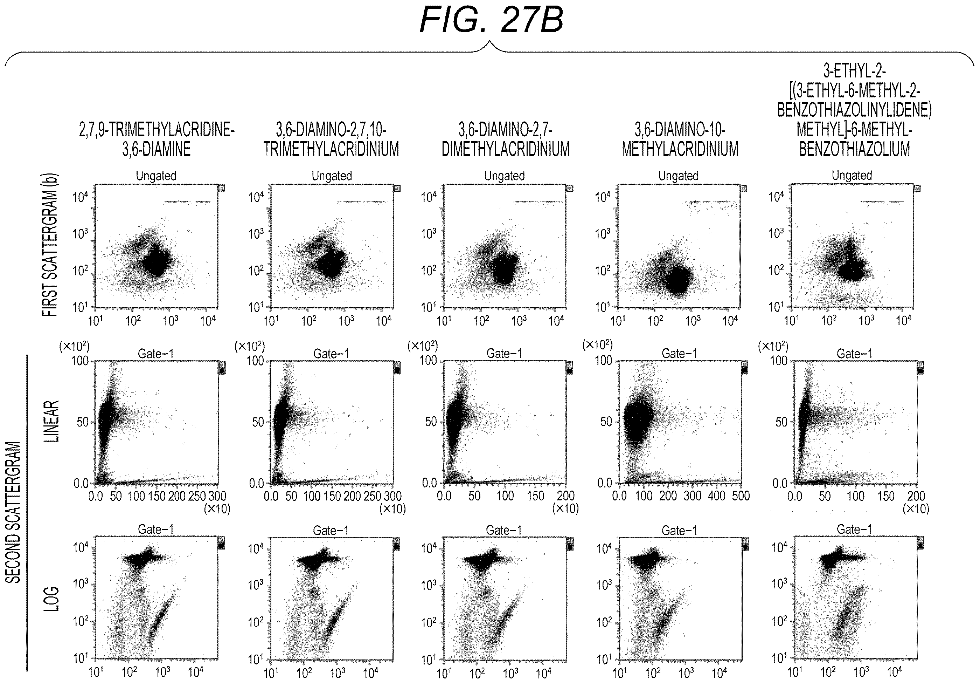

Figs. 26A and 26B contain the first and second scattergrams of measurement samples prepared from the low-PLT specimen 1, with use of ten species of the aforementioned fluorescent dyes. Figs. 27A and 27B contain the first and second scattergrams of measurement samples prepared from the low-PLT specimen 2, with use of 10 species of the aforementioned fluorescent dyes. With reference to these drawings, white blood cell was found to be distinguishable from the blood cells other than white blood cell on the first scattergrams (a) and (b), whichever fluorescent dye was used. The second scattergram in linear scale was found to contain the individual populations of mature red blood cell, reticulocyte, and platelet. In the second scattergram in logarithmic scale, the platelet population and the debris population were found apart from each other, thus enabling discrimination between both populations. As can be seen, reticulocyte and platelet were proved to be successfully classified, with use of various fluorescent dyes that belong to the acridine-based compound and the cyanine-based compound. It was also found that platelet and debris are discriminable.

### Example 4: Analysis of Measurement Sample that Contains Platelet Aggregation

Measurement samples were prepared from blood specimens that contain platelet aggregation, and platelet aggregation in the measurement samples was detected with an analyzer.

### (1) Measurement Samples

Blood specimens from healthy subjects, and blood specimens determined to be positive with respect to the platelet aggregation by visual observation under a microscopic (hereinafter also referred to as "PLTc-negative specimen" and "PLTc-positive specimen", respectively), were selected. From these blood specimens, the measurement samples were prepared as described previously. An ethylene glycol solution of 2,7,9-trimethylacridine-3,6-diamine was used as the staining reagent. The final concentration of the fluorescent dye in the measurement sample was 1.0 ppm.

### (2) Analysis

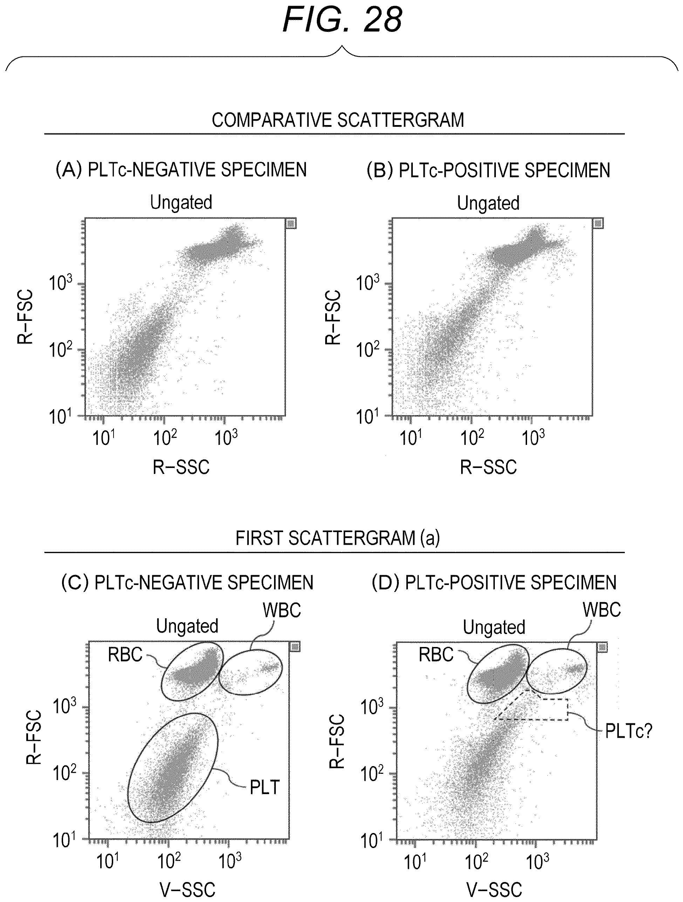

The individual measurement samples were measured with an analyzer (prototype modified from XN-20), to acquire the fluorescence information, the first scattered light information, the second scattered light information, and the third scattered light information. The fluorescence information, the first scattered light information, and the second scattered light information were the same as those in Example 1. The third scattered light information corresponded to the side-scattered light intensity obtained by irradiating the particles in the measurement sample with a red laser (also referred to as "forward-scattered light intensity (red)" or "R-SSC", hereinafter). The first scattergrams (a) of the individual measurement samples were prepared, in the same manner as in Example 1. For comparison, scattergrams with the side-scattered light intensity (red) plotted on the abscissa, and with the forward-scattered light intensity (red) plotted on the ordinate, were prepared. The individual scattergrams are illustrated in (A) to (D) in Fig. 28. In the drawing, "PLTc?" stands for particle suspected of forming platelet aggregation.

Referring now to the comparative scattergram of the PLTc-negative specimen named (A) in Fig. 28, the platelet population was identifiable, meanwhile white blood cell and red blood cell appeared as overlapped. White blood cells and red blood cell, therefore, could not be classified. Referring now to the comparative scattergram of the PLTc-positive specimen named (B) in Fig. 28, observed was a particle group that distributes to extend from the platelet population towards the population where white blood cell and red blood cell overlap. Whether or not the particle group represents red blood cell or platelet aggregation, however, could not be determined. As can be seen, platelet aggregation was hardly detectable with reference to the side-scattered light intensity and the forward-scattered light intensity obtained only with the red laser.

Referring now to the first scattergram (a) of the PLTc-negative specimen named (C) in Fig. 28, the white blood cell population, the red blood cell population, and the platelet population were clearly classified on. Referring now to (D) in Fig. 28, also the first scattergram (a) of the PLTc-positive specimen clearly classified the white blood cell population, the red blood cell population, and the platelet population. On the scattergram named (D), there appeared a particle group that was not observed on the scattergram named (C) in an area surrounded by the broken line. Platelet aggregation is larger in size than normal platelet, and this makes the forward-scattered light intensity higher. The particle group in the area surrounded by the broken line was thus discriminable as the platelet aggregation. On the scattergram named (D), a flag "PLTc?" was used to indicate appearance of particle suspected of forming platelet aggregation. On the first scattergrams (a), appearance of platelet aggregation was clearly recognized, since the red blood cell population appeared in the areas where V-SSC is lower than that in the comparative scattergrams. As can be seen, platelet aggregation was proved to be detectable, with reference to the side-scattered light intensity obtained by the blue-violet laser, and the forward-scattered light intensity obtained by the red laser.

### Example 5: Classification and Counting of White Blood Cell

In Example 1 to 3, the blood cells other than white blood cell were sorted on the first scattergram, meanwhile in Example 5, white blood cell was sorted on the first scattergram. White blood cell was further classified and counted.

### (1) Measurement Samples

The measurement samples were prepared from the blood specimens from healthy persons, as described above. An ethylene glycol solution of 2,7,9-trimethylacridine-3,6-diamine was used as the staining reagent. The final concentration of the fluorescent dye in the measurement sample was 1.0 ppm.

### (2) Analysis

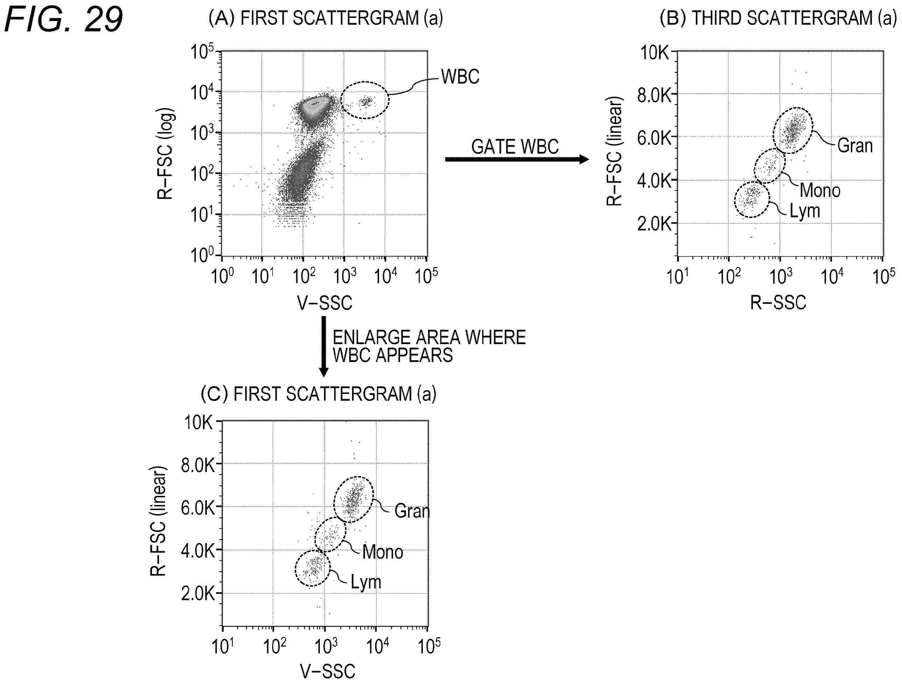

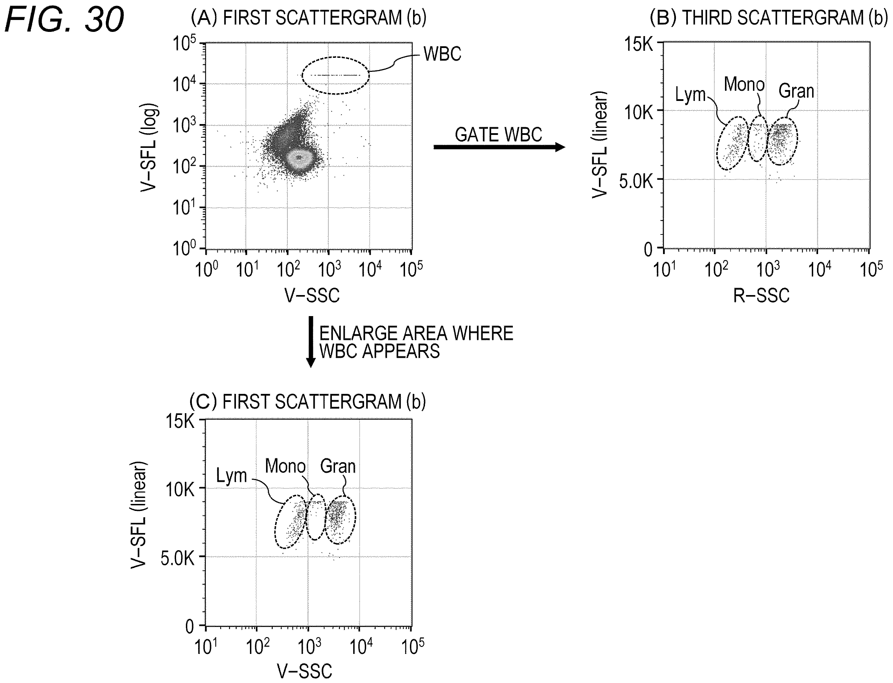

The individual measurement samples were measured with an analyzer (prototype modified from XF-1600), to acquire the fluorescence information, the first scattered light information, the second scattered light information, and the third scattered light information. The fluorescence information, the first scattered light information, and the second scattered light information were the same as those in Example 1. The third scattered light information was the same as that in Example 4. Example 5 employed prolonged measurement time with the analyzer, and increased count of the particles to be analyzed. The first scattergrams (a) and (b) of the individual measurement samples were prepared, in the same manner as in Example 1. These scattergrams are illustrated in (A) in Fig. 29 and (A) in Fig. 30. As can be seen in these drawings, white blood cell was found to be distinguishable from the blood cells other than white blood cell, on the first scattergrams (a) and (b). On the first scattergrams (a) and (b), the white blood cell count was successfully obtained by counting the particles that were identified as white blood cell.

Next, the white blood cell that appeared on the first scattergrams (a) and (b) was sorted. The white blood cell sorted from the first scattergram (a) was plotted on a scattergram with the side-scattered light intensity (red) plotted on the abscissa, and with the forward-scattered light intensity (red) plotted on the ordinate (also referred to as "third scattergram (a)", hereinafter). Also the white blood cell sorted from the first scattergram (b) was plotted on a scattergram with the side-scattered light intensity (red) plotted on the abscissa, and with the fluorescence intensity (blue-violet) plotted on the ordinate (also referred to as "third scattergram (b)", hereinafter). The scattergrams are given by (B) in Fig. 29, and (B) in Fig. 30. In the drawings, "Gran" stands for granulocyte, "Mono" stands for monocyte, and "Lym" stands for lymphocyte. Referring now to the third scattergrams, white blood cell was proved to be classified into the subpopulations, with reference to the side-scattered light intensity and the forward-scattered light intensity obtained only by the red laser. Also granulocyte, monocyte, and lymphocyte were successfully counted, by counting the particles contained in the individual subpopulations.

Whether or not white blood cell can be classified into subpopulations on the individual first scattergrams, without sorting white blood cell, was then examined. Enlarged views of the areas where white blood cell appeared in the first scattergrams (a) and (b) are given in (C) of (C) in Fig. 29 and (C) in Fig. 30, respectively. Referring now to the drawings, white blood cell was proved to be classified into the subpopulations, by enlarging the areas where white blood cell appeared on the first scattergrams (a) and (b). Also granulocyte, monocyte, and lymphocyte were successfully counted, by counting the particles contained in the individual subpopulations.

### Example 6: Classification and Counting of White Blood Cell

The white blood cell counts acquired on the first scattergrams (a) and (b) were compared with the white blood cell count acquired by the prior method, and a correlation in between was examined. In the prior method, the measurement sample was measured with the automated hematology analyzer XN-20 (Sysmex Corporation), and white blood cell was counted by analysis with a WDF channel and WNR channel.

### (1) Measurement Samples

From 24 specimens that include normal specimens and abnormal specimens, the measurement samples were prepared as described above. The normal specimens were blood specimens from healthy persons. The abnormal specimens were found to have abnormality in the blood cell component (excluding specimens having high contents of nucleated red blood cell). An ethylene glycol solution of 2,7,9-trimethylacridine-3,6-diamine was used as the staining reagent. The final concentration of the fluorescent dye in the measurement sample was 1.0 ppm. Each measurement sample to be analyzed with the WDF channel of XN-20 was prepared on XN-20, with use of each of the aforementioned 24 specimens, Fluorocell (registered trademark) WDF (Sysmex Corporation) as a staining reagent, and Lysercell (registered trademark) WDF (Sysmex Corporation) as a hemolysis reagent. Each measurement sample to be analyzed with the WNR channel of XN-20 was prepared on XN-20, with use of each of the aforementioned 24 specimens, Fluorocell (registered trademark) WNR (Sysmex Corporation) as a staining reagent, and Lysercell (registered trademark) WNR (Sysmex Corporation) as a hemolysis reagent.

### (2) Analysis

The individual measurement samples were measured with an analyzer (a prototype modified from XF-1600), to acquire the first scattered light information, the second scattered light information, and the fluorescence information. The individual information were the same as those in Example 1. The first scattergrams (a) and (b) of the individual measurement samples were prepared, in the same manner as in Example 1. White blood cell was counted on the individual scattergrams (a) and (b), in the same manner as in Example 5. In the prior method, each measurement sample was measured with XN-20, and white blood cell was counted with the WDF channel and the WNR channel. XN-20 herein was equipped with a semiconductor laser light source that emits red (639 nm) light, a detector for red forward-scattered light, a detector for red side-scattered light, and a detector for fluorescence (660 nm or longer) excited by the red light. In the measurement with XN-20, the fluorescence intensity obtained by irradiating the particles in the measurement sample with a red laser (hereinafter, also referred to as "fluorescence intensity (red)"), the side-scattered light intensity (red), and the forward-scattered light intensity (red) were acquired. With the WDF channel, blood cells were analyzed with reference to the side- scattered light intensity (red) and the fluorescence intensity (red). With the WNR channel, blood cells were analyzed with reference to the forward-scattered light intensity (red) and the fluorescence intensity (red).

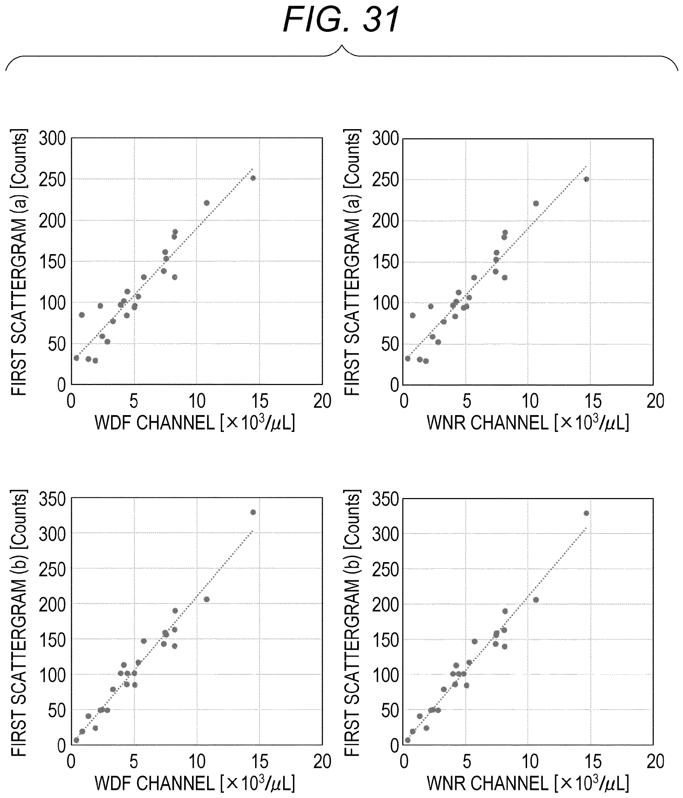

Correlations of the white blood cell count acquired from each of the first scattergrams (a) and (b), with the white blood cell count acquired from each of the WDF channel and the WNR channel were examined. Results are illustrated in Fig. 31. Correlation coefficients (r) regarding the white blood cell count, between each of the first scattergrams (a) and (b), and each of the and the WDF channel and the WNR channel, are summarized in Table 1. As can be seen from Fig. 31 and Table 1, the white blood cell counts acquired from each of the first scattergrams (a) and (b) demonstrated good correlations with the white blood cell count acquired from each of the WDF channel and the WNR channel. The method for analyzing blood cells of this embodiment, although without use of a hemolysis reagent, was proved to count white blood cell with an accuracy comparable to that of the prior method with use of the hemolysis reagent.

**[Table 1]**

| | WDF channel | WNR channel |
|---|---|---|
| First scattergram (a) | 0.94 | 0.94 |
| First scattergram (b) | 0.98 | 0.98 |

### Example 7: Analysis of Platelet

The platelet population classified on the second scattergram was analyzed, to acquire values of various indexes regarding platelet.

### (1) Measurement Samples

The measurement samples were prepared from the blood specimens from healthy persons, as described above. An ethylene glycol solution of 2,7,9-trimethylacridine-3,6-diamine was used as the staining reagent. The final concentration of the fluorescent dye in the measurement sample was 1.0 ppm.

### (2) Analysis

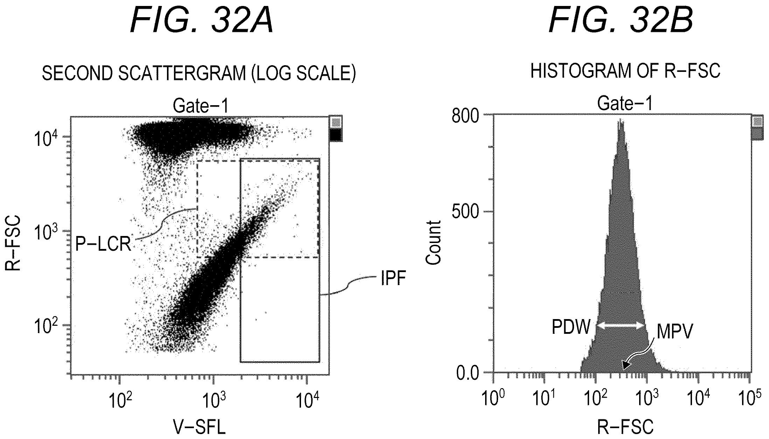

The individual measurement samples were measured with an analyzer (a prototype modified from XN-20), to acquire the fluorescence information, the first scattered light information, and the second scattered light information. The individual information were the same as those in Example 1. The first scattergram (a) was created, to sort the blood cells other than white blood cell, in the same manner as in Example 1. The sorted blood cells were plotted on the second scattergram (in logarithmic scale). An example of the second scattergram is illustrated in (A) in Fig. 32. Platelet was identified on the second scattergram. The particles identified as platelet were counted, to acquire the platelet count. The platelet population on the second scattergram was sorted, and used to create a histogram with reference to the forward-scattered light intensity (red) and the particle count. An example of the histogram is illustrated in (B) in Fig. 32.

Referring now to distribution of platelet on the second scattergram named (A) in Fig. 32, the immature platelet fraction (IPF) and the percentage of large platelet (P-LCR) were acquired. With reference to the histogram named (B) in Fig. 32, the mean platelet volume (MPV) and the platelet distribution width (PDW) were acquired. Exemplary values acquired for the individual indices are summarized in Table 2. As can be seen, the method for analyzing blood cells of this embodiment was proved to acquire various indices regarding platelet.

**[Table 2]**

| Item | Value | Unit |
|---|---|---|
| MPV | 9.3 | fL |
| PDW | 13.6 | fL |
| P-LCR | 17.9 | % |
| IPF | 1.9 | % |

## Claims

1. A method for analyzing blood cells, the method comprising:
acquiring first scattered light information, second scattered light information, and fluorescence information generated by irradiating a measurement sample that contains a particle stained with a fluorescent dye with a light of a first wavelength and a light of a second wavelength;
identifying blood cells other than white blood cell from the particles in the measurement sample, with reference to the first scattered light information and the second scattered light information, and/or, with reference to the first scattered light information and the fluorescence information; and
classifying the blood cells other than white blood cell into reticulocyte and platelet, with reference to the fluorescence information and the second scattered light information,
the first wavelength being 315 nm or longer and 600 nm or shorter, and the second wavelength being 610 nm or longer and 750 nm or shorter.

2. The method according to claim 1, wherein
the first scattered light information is information related to side-scattered light generated from the particle upon irradiation of the measurement sample with the light of the first wavelength,
the second scattered light information is information related to forward-scattered light generated from the particle upon irradiation of the measurement sample with the light having the second wavelength, and
the fluorescence information is information related to fluorescence generated from the fluorescent dye on the particle upon irradiation of the measurement sample with the light of the first wavelength.

3. The method according to claim 1 or 2, wherein in the step of classifying, the blood cells other than white blood cell are classified into reticulocyte, platelet, and mature red blood cell.

4. The method according to any of claims 1 to 3, wherein the first scattered light information is an intensity of first side-scattered light from the particle irradiated with the light of the first wavelength, the second scattered light information is an intensity of forward-scattered light from the particle irradiated with the light of the second wavelength, and the fluorescence information is an intensity of fluorescence from the fluorescent dye on the particle irradiated with the light of the first wavelength; and
in the step of identifying, the blood cells other than white blood cell are identified, with reference to the first side-scattered light intensity, and the forward-scattered light intensity or the fluorescence intensity.

5. The method according to claim 4, wherein in the step of identifying, a scattergram is created, with reference to the first side-scattered light intensity, and the forward-scattered light intensity or the fluorescence intensity, and the blood cells other than white blood cell are identified on the scattergram.

6. The method according to claim 4 or 5, wherein in the step of identifying:
the particles in the measurement sample are classified into a first particle population and a second particle population, with reference to the first side-scattered light intensity and the forward-scattered light intensity; and,
the second particle population is a population with higher first side-scattered light intensity and/or the forward-scattered light intensity than the first particle population,
the first particle population is identified as the blood cells other than white blood cell.

7. The method according to claim 4 or 5, wherein in the step of identifying:
the particles in the measurement sample are classified into a first particle population and a second particle population, with reference to the first side-scattered light intensity and the fluorescence intensity; and,
the second particle population is a population with higher first side-scattered light intensity and/or the fluorescence intensity than the first particle population,
the first particle population is identified as the blood cells other than white blood cell.

8. The method according to any of claims 1 to 7, wherein the second scattered light information is an intensity of the forward-scattered light from the particle irradiated with the light of the second wavelength, the fluorescence information is an intensity of the fluorescence from the particle irradiated with the light of the first wavelength, and
in the step of classifying, the blood cells other than white blood cell are classified into reticulocyte and platelet, with reference to the fluorescence intensity and the forward-scattered light intensity.

9. The method according to claim 8, wherein in the step of classifying, a scattergram is created with reference to the fluorescence intensity and the forward-scattered light intensity, and the blood cells other than white blood cell are classified into reticulocyte and platelet, on the scattergram.

10. The method according to claim 8 or 9, wherein in the step of classifying:
the blood cells other than white blood cell are classified into a third particle population, a fourth particle population, and a fifth particle population, with reference to the fluorescence intensity and the forward-scattered light intensity; and,
the third particle population is a population with higher fluorescence intensity than the fifth particle population, and
the fourth particle population is a population with lower forward-scattered light intensity than the third particle population,
the third particle population is identified as a reticulocyte population, and the fourth particle population as a platelet population.

11. The method according to claim 10, wherein the fifth particle population is identified as a mature red blood cell population.

12. The method according to any of claims 1 to 10, wherein in the step of identifying, white blood cell is further identified, and the white blood cell is classified into subpopulations.

13. The method according to claim 12, wherein the white blood cell is identified with reference to the first scattered light information and the second scattered light information, and/or, with reference to the first scattered light information and the fluorescence information, and the white blood cell is classified into subpopulations.

14. The method according to claim 13, wherein the first scattered light information is an intensity of the first side-scattered light from the particle irradiated with the light of the first wavelength, the second scattered light information is an intensity of the forward-scattered light from the particle irradiated with the light of the second wavelength, the fluorescence information is an intensity of fluorescence from the fluorescent dye on the particle irradiated with the light of the first wavelength; and
the white blood cell is identified with reference to the first side-scattered light intensity, and the forward-scattered light intensity or the fluorescence intensity, and is classified into subpopulations.

15. The method according to claim 14, wherein a scattergram with reference to the first side-scattered light intensity and the forward-scattered light intensity, and/or, a scattergram with reference to the first side-scattered light intensity and the fluorescence intensity is prepared and the white blood cell is identified and classified into on the subpopulations on the scattergram.

16. The method according to claim 12, wherein in the step of acquiring, a third scattered light information is further acquired; and
the third scattered light information is information related to a side-scattered light generated from the particle upon irradiation of the measurement sample with the light of the second wavelength,
the white blood cell is identified with reference to the first scattered light information and the second scattered light information, and/or, with reference to the first scattered light information and the fluorescence information; and
the white blood cell is classified into the subpopulations with reference to the second scattered light information and the third scattered light information, and/or, with reference to the fluorescence information and the third scattered light information.

17. The method according to claim 16, wherein the second scattered light information is an intensity of the forward-scattered light from the particle irradiated with the light of the second wavelength, the third scattered light information is an intensity of the second side-scattered light from the particle irradiated with the light of the second wavelength, and the fluorescence information is an intensity of fluorescence from the fluorescent dye on the particle irradiated with the light of the first wavelength; and
the white blood cell is classified into subpopulations, with reference to the second side-scattered light intensity, and the forward-scattered light intensity or the fluorescence intensity.

18. The method according to claim 17, wherein a scattergram is created with reference to the second side-scattered light intensity and the forward-scattered light intensity, and/or, a scattergram is created with reference to the second side-scattered light intensity and the fluorescence intensity, and the white blood cell is classified into subpopulations on the scattergram.

19. The method according to any of claims 12 to 18, wherein the subpopulations comprise at least two populations selected from lymphocyte population, monocyte population, and granulocyte population.

20. The method according to any of claims 1 to 19, wherein the identifying further comprises acquiring information that indicates presence or absence of platelet aggregation, with reference to the first scattered light information and the second scattered light information.

21. The method according to claim 20, wherein the first scattered light information is the intensity of the side-scattered light from the particle irradiated with the light of the first wavelength, the second scattered light information is the intensity of the forward-scattered light from the particle irradiated with the light of the second wavelength; and
in the step of identifying, the blood cells other than white blood cell are identified, with reference to the forward-scattered light intensity and the side-scattered light intensity,
the blood cells other than white blood cell are classified into a red blood cell-containing population and a platelet-containing population with reference to the forward-scattered light intensity, and,
information that indicates presence of the platelet aggregation is acquired, if the platelet-containing population contains a particle that exhibits a forward-scattered light intensity equal to or above a threshold value.

22. The method according to claim 21, wherein in the step of identifying, a scattergram is created with reference to the forward-scattered light intensity and the side-scattered light intensity; the particles in the measurement sample are identified as a red blood cell-containing population, a platelet-containing population, and white blood cell on the scattergram; and
information that indicates presence of the platelet aggregation is acquired, if the platelet-containing population contains a particle that exhibits a forward-scattered light intensity equal to or above a threshold value.

23. The method according to any of claims 1 to 22, wherein in the step of classifying, the platelet is further classified into mature platelet and immature platelet.

24. The method according to any of claims 1 to 23, wherein in the step of classifying, a large platelet is identified from the platelet.

25. The method according to any of claims 1 to 24, further comprising estimating a platelet distribution width and/or a mean platelet volume.

26. The method according to any of claims 1 to 25, wherein the fluorescent dye is selected from the group consisting of acridine-based compound, cyanine-based compound, and styryl-based compound.

27. The method according to claim 26, wherein the acridine-based compound is represented by formula (I) below: 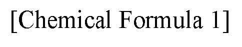 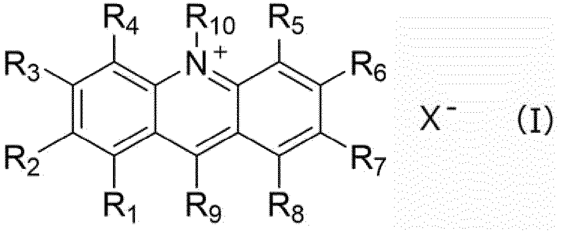 (where, each of R₁ to R₁₀ independently is a hydrogen atom, -NH₂, -(CH₂)ₙ-NR₁₁R₁₂, - NH-R₁₃-NR₁₁R₁₂, -O-R₁₁, -COOH, a halogen, a phenyl group optionally having a substituent, an alkyl group having 1 to 18 carbon atoms, or an acylamino group, provided that at least one of R₃, R₆, and R₉ independently is -NH₂, -(CH₂)ₙ-NR₁₁R₁₂, or phenyl group substituted with -NH₂;
each of R₁₁ and R₁₂, identically or differently, is a hydrogen atom or alkyl group having 1 to 18 carbon atoms;
R₁₃ is an alkyl group having 1 to 6 carbon atoms;
n is an integer of 0 to 6; and
X⁻ is a counter ion).

28. The method according to claim 26 or 27, wherein the cyanine-based compound is represented by formula (II) below: 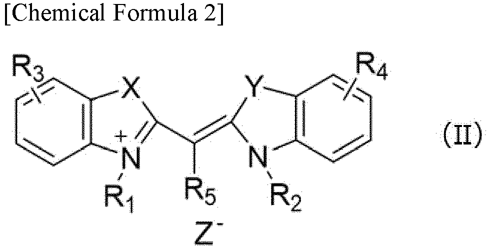 (where, each of R₁ and R₂, identically or differently, is an alkyl group having 1 to 18 carbon atoms, an alkenyl group having 3 to 20 carbon atoms, an alkyl group having 1 to 18 carbon atoms and a hydroxy group, an alkyl group having 1 to 18 carbon atoms and a carboxy group, an alkyl group having 1 to 18 carbon atoms and a sulfo group, a halogen, -(CH₂)ₘ-NR₆R₇, -(CH₂)ₙ-O-R₈, or a benzyl group optionally having a substituent;
each of R₃ and R₄, identically or differently, is a hydrogen atom, a halogen, an alkyl group having 1 to 18 carbon atoms, a phenyl group optionally having a substituent, an alkoxy group having 1 to 6 carbon atoms, or -(CH₂)ₙ-O-R₈;
R₅ is a hydrogen atom, a halogen, an alkyl group having 1 to 6 carbon atoms, an alkyl group having 1 to 6 carbon atoms and a hydroxy group, an alkyl group having 1 to 6 carbon atoms and a carboxy group, an alkyl group having 1 to 6 carbon atoms and a sulfo group, a phenyl group optionally having a substituent, a benzyl group optionally having a substituent, or -(CH₂)ₙ-O-R₆;
each of R₆ and R₇, identically or differently, is a hydrogen atom, or an alkyl group having 1 to 18 carbon atoms;
R₈ is a hydrogen atom, an alkyl group having 1 to 18 carbon atoms, or a phenyl group optionally having a substituent;
m is an integer of 1 to 18, and n is an integer of 1 to 6;
each of X and Y, identically or differently, is a sulfur atom, an oxygen atom, a selenium atom, or CR₉R₁₀;
each of R₉ and R₁₀, identically or differently, is an alkyl group having 1 to 3 carbon atoms; and
Z⁻ is a counter ion).

29. The method according to any of claims 26 to 28, wherein the styryl-based compound is represented by formula (III) below: 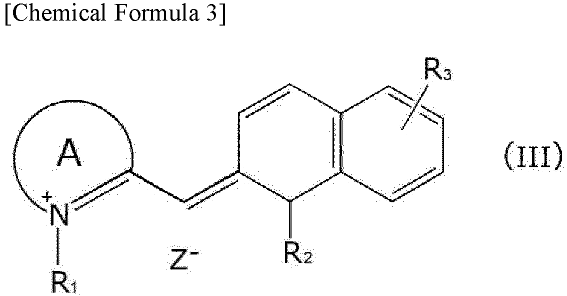 (where, R₁ is an alkyl group having 1 to 18 carbon atoms, an alkenyl group having 3 to 20 carbon atoms, an alkyl group having 1 to 18 carbon atoms and a hydroxy group, an alkyl group having 1 to 18 carbon atoms and a carboxy group, an alkyl group having 1 to 18 carbon atoms and a sulfo group, a halogen, -(CH₂)ₚ-NR₅R₆, -(CH₂)_{q}-O-R₇, or a benzyl group optionally having a substituent;
each of R₂ and R₃, identically or differently, is a hydrogen atom, a halogen, an alkyl group having 1 to 6 carbon atoms, an alkyl group having 1 to 18 carbon atoms, a phenyl group optionally having a substituent, or an alkoxy group having 1 to 6 carbon atoms; 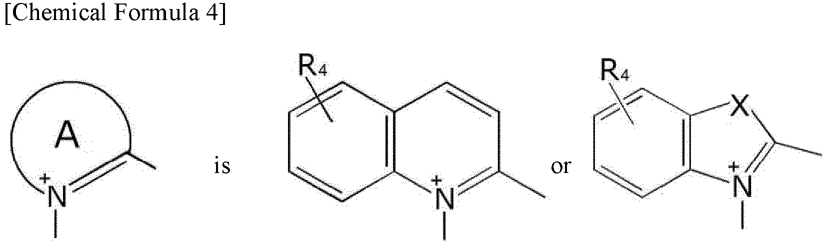
R₄ is a hydrogen atom, a halogen, an alkyl group having 1 to 18 carbon atoms, a phenyl group optionally having a substituent, an alkoxy group having 1 to 6 carbon atoms, or -(CH₂)ₘ-O-R₇;
each of R₅ and R₆, identically or differently, is a hydrogen atom, or an alkyl group having 1 to 18 carbon atoms;
R₇ is a hydrogen atom, an alkyl group having 1 to 18 carbon atoms, or a phenyl group optionally having a substituent;
X is a sulfur atom, an oxygen atom, a selenium atom, or CR₈R₉;
each of R₈ and R₉, identically or differently, is an alkyl group having 1 to 3 carbon atoms;
m is an integer of 1 to 18; and
Z⁻ is a counter ion), or represented by formula (IV) below: 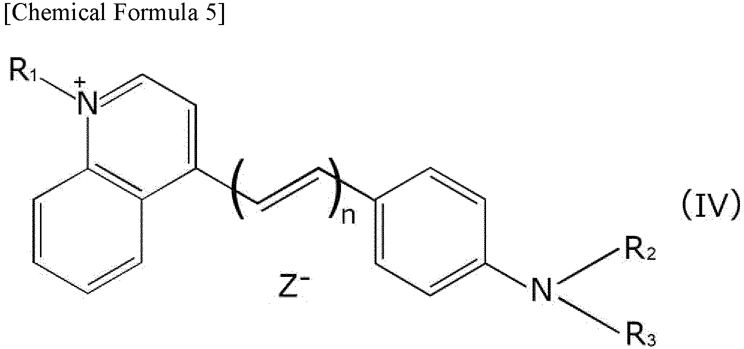 (where, R₁ and Z⁻ are same as those described above,
each of R₂ and R₃, identically or differently, is a hydrogen atom, a halogen, an alkyl group having 1 to 18 carbon atoms, a phenyl group optionally having a substituent, or an alkoxy group having 1 to 6 carbon atoms, or, R₂ and/or R₃ forms an N-containing heterocycle together with a benzene ring to which the N is bound, and
n is an integer of 0 to 2).

30. The method according to any of claims 1 to 29, wherein the first wavelength is 315 nm or longer and 490 nm or shorter.

31. A reagent for blood cell analysis, the reagent comprising a fluorescent dye selected from the group consisting of acridine-based compound, cyanine-based compound, and styryl-based compound, and used for the method according to claim 1.
